# EUROPEAN PATENT APPLICATION

(11) **EP 3 988 116 A1**
(43) Date of publication of application: **27.04.2022**
(21) Application number: 20382923.9
(22) Date of filing: 23.10.2020
(51) Int. Cl.: A61K 39/395, A61P 35/02, G01N 33/50, C12Q 1/6886

(54) **METHODS AND COMPOSITIONS FOR THE TREATMENT OF HEMATOLOGIC MALIGNANCIES**

(71) Applicant: Consejo Superior De Investigaciones Científicas (CSIC), 28006 Madrid (ES); Fundación Instituto de Estudios Ciencias de la Salud de Castilla y León (IECSCYL-IBSAL), 37007 Salamanca (ES)
(72) Inventor: SÁNCHEZ GARCÍA, Isidro, 37007 Salamanca (ES); VICENTE- DUEÑAS, Carolina, 37007 Salamanca (ES)
(74) Representative: Pons

(57) **Abstract**

The present invention relates to Interleukin 6 (IL-6) inhibitors for use in the treatment and/or prevention of hematologic malignancies comprising deficiencies in the B-cell transcription factor gene PAX5 in a subject, as well as to an *in vitro* method for for diagnosing hematologic malignancies in a subject, or for designing a personalized therapy for a subject with hematologic malignancies.

SEQUENCE LISTING

<110> Centro Superior de Investigaciones Científicas

<120> METHODS AND COMPOSITIONS FOR THE TREATMENT OF HEMATOLOGIC MALIGNANCIES

<130> 1641.1622

<160> 17

<170> BiSSAP 1.3.6

<210> 1
<211> 4799
<212> DNA
<213> Homo sapiens

<400> 1
attctgccct cgagcccacc gggaacgaaa gagaagctct atctcccctc caggagccca 60

gctatgaact ccttctccac aagtaagtgc aggaaatcct tagccctgga actgccagcg 120

gcggtcgagc cctgtgtgag ggaggggtgt gtggcccagg gagggctggc gggcggccag 180

cagcagaggc aggctcccag ctgtgctgtc agctcacccc tgcgctcgct cccctccggc 240

acaggcgcct tcggtccagt tgccttctcc ctggggctgc tcctggtgtt gcctgctgcc 300

ttccctgccc cagtaccccc aggagaagat tccaaagatg tagccgcccc acacagacag 360

ccactcacct cttcagaacg aattgacaaa caaattcggt acatcctcga cggcatctca 420

gccctgagaa aggaggtggg taggcttggc gatggggttg aagggcccgg tgcgcatgcg 480

ttccccttgc ccctgcgtgt ggccgggggc tgcctgcatt aggaggtctt tgctgggttc 540

tagagcactg tagatttgag gccaacgggg ccgactagac tgacttctgt atttatcctt 600

tgctggtgtc aggaagttcc tttcctttct ggaaaatgca gaatgggtct gaaatccatg 660

cccacctttg gcatgagctg agggttattg cttctcaggg cttccttttc cctttccaaa 720

aaattaggtc tgtgaagctc ctttttgtcc cccgggcttt ggaaggacta gaaaagtgcc 780

acctgaaagg catgttcagc ttctcagagc agttgcagta ctttttggtt atgtaaactc 840

aatggctagg attcctcaaa gccattccag ctaagattca tacctcagag cccaccaaag 900

tggcaaatca taaataggtt aaagcatctc cccactttca atgcaaggta ttttggtcct 960

gtttggtaga aagaaaagaa cacaggaggg gagattggga gcccacactc gaattctggt 1020

tctgccaaac cagccttgtg atcttgggta aattccctac cacctctgga ctccatcagt 1080

aaaattgggc gtggactagg tgatctcata gatccttcct gctggaacat tctatggctt 1140

gaattatatt ctcctaatta ttgtcaaaat tgctgttatt aagtatctac tgtgtgccag 1200

gcactttaaa taaatattgt gtctaatctt caaaacaaat ttgcaaggaa ggtttttgga 1260

gataaggaaa ctgagactca ggattaagta acacacctaa agtcacaggt gagcttggaa 1320

ctgaacccaa gtgtgccccc actccactgg aatttgcttg ccaggatgcc aatgagttgt 1380

agcttcattt ttcttagaga ctttcctggc tgtggttgaa caatgaaaag gccctctagt 1440

ggtgtttgtt ttagggacac ttaggtgata acaattctgg tattctttcc cagacatgta 1500

acaagagtaa catgtgtgaa agcagcaaag aggcactggc agaaaacaac ctgaaccttc 1560

caaagatggc tgaaaaagat ggatgcttcc aatctggatt caatgaggta ccaacttgtc 1620

gcactcactt ttcactattc cttaggcaaa acttctccct cttgcatgca gtgcctgtat 1680

acatatagat ccaggcagca acaaaaagtg ggtaaatgta aagaatgtta tgtaaatttc 1740

atgaggaggc caacttcaag cttttttaaa ggcagtttat tcttggacag gtatggccag 1800

agatggtgcc actgtggtga gattttaaca actgtcaaat gtttaaaact cccacaggtt 1860

taattagttc atcctgggaa aggtactctc agggcctttt ccctctctgg ctgcccctgg 1920

cagggtccag gtctgccctc cctccctgcc cagctcattc tccacagtga gataacctgc 1980

actgtcttct gattatttta taaaaggagg ttccagccca gcattaacaa gggcaagagt 2040

gcaggaagaa catcaagggg gacaatcaga gaaggatccc cattgccaca ttctagcatc 2100

tgttgggctt tggataaaac taattacatg gggcctctga ttgtccagtt atttaaaatg 2160

gtgctgtcca atgtcccaaa acatgctgcc taagaggtac ttgaagttct ctagaggagc 2220

agagggaaaa gatgtcgaac tgtggcaatt ttaacttttc aaattgattc tatctcctgg 2280

cgataaccaa ttttcccacc atctttcctc ttaggagact tgcctggtga aaatcatcac 2340

tggtcttttg gagtttgagg tatacctaga gtacctccag aacagatttg agagtagtga 2400

ggaacaagcc agagctgtgc agatgagtac aaaagtcctg atccagttcc tgcagaaaaa 2460

ggtgggtgtg tcctcattcc ctcaacttgg tgtgggggaa gacaggctca aagacagtgt 2520

cctggacaac tcagggatgc aatgccactt ccaaaagaga aggctacacg taaacaaaag 2580

agtctgagaa atagtttctg attgttattg ttaaatcttt ttttgtttgt ttggttggtt 2640

ggctctcttc tgcaaaggac atcaataact gtattttaaa ctatatatta actgaggtgg 2700

attttaacat caatttttaa tagtgcaaga gatttaaaac caaaggcggg ggggcgggca 2760

gaaaaaagtg catccaactc cagccagtga tccacagaaa caaagaccaa ggagcacaaa 2820

atgattttaa gattttagtc attgccaagt gacattcttc tcactgtggt tgtttcaatt 2880

ctttttccta ccttttacca gagagttagt tcagagaaat ggtcagagac tcaagggtgg 2940

aaagaggtac caaaggcttt ggccaccagt agctggctat tcagacagca gggagtagac 3000

ttgctggcta gcatgtggag gagccaaagc tcaataagaa ggggcctaga atgaaaccct 3060

tggtgctgat cctgcctctg ccatttctac ttaagccagg gtttctcata tgttaacatg 3120

catgggaatt ccctgggcat cttcttgtgg tgtggagtct gacttagcaa gcctcgggtg 3180

ggtttgaggg tcaaatttct accaggctta tatccctggt gatgctgcag aattccagga 3240

ccacacttgg aggtttaagg ccttccacaa gttacttatc ccatatggtg ggtctatgga 3300

aaggtgtttc ccagtcctct ttacaccacc ggatcagtgg tctttcaaca gatcctaaag 3360

ggatggtgag agggaaactg gagaaaagta tcagatttag aggccactga agaacccata 3420

ttaaaatgcc tttaagtatg ggctcttcat tcatatacta aatatgaact atgtgccagg 3480

cattatttca tatgacagaa tacaaacaaa taagatagtg atgctggtca ggcttggtgg 3540

ctcatgcctg tattccctaa actttgggag cctaaggtga gaactccttg aactcctaag 3600

gccaggagtt caagaccagc ctggataaca tagcaagacc ccatctctac aaaaaaccaa 3660

aaccaaacaa acaaaaatga tagtggtgct tccctcagga tgcttgtggt ctaatgggag 3720

acagaacagc aaagggatga ttagaagttg gttgctgtga gccaggcaca gtgctgatat 3780

aatcccagcg ctatgggagg ctgaggtggg tggatcattt gaggccagga gtttaagacc 3840

agcctggtca acatggtaaa accccatctc tacttaaaaa tacaaaaaag ttagccaggc 3900

atggtggcat acacctgtaa cccagctact caggaggctg aggcacatga atcacttgaa 3960

cccaggaggc agaggttgct gtgcaccact gcactccagc ctgggtgaca gaacgagacc 4020

ttgactcaaa aaaaaaaaaa agaagtttgt tgctatggaa gggtcctact cagagcaggc 4080

accccagtta atctcattca ccccacattt cacatttgaa catcatccca tagcccagag 4140

catccctcca ctgcaaagga tttattcaac atttaaacaa tcctttttac tttcattttc 4200

cttcaggcaa agaatctaga tgcaataacc acccctgacc caaccacaaa tgccagcctg 4260

ctgacgaagc tgcaggcaca gaaccagtgg ctgcaggaca tgacaactca tctcattctg 4320

cgcagcttta aggagttcct gcagtccagc ctgagggctc ttcggcaaat gtagcatggg 4380

cacctcagat tgttgttgtt aatgggcatt ccttcttctg gtcagaaacc tgtccactgg 4440

gcacagaact tatgttgttc tctatggaga actaaaagta tgagcgttag gacactattt 4500

taattatttt taatttatta atatttaaat atgtgaagct gagttaattt atgtaagtca 4560

tatttatatt tttaagaagt accacttgaa acattttatg tattagtttt gaaataataa 4620

tggaaagtgg ctatgcagtt tgaatatcct ttgtttcaga gccagatcat ttcttggaaa 4680

gtgtaggctt acctcaaata aatggctaac ttatacatat ttttaaagaa atatttatat 4740

tgtatttata taatgtataa atggttttta taccaataaa tggcatttta aaaaattca 4799

<210> 2
<211> 212
<212> PRT
<213> Homo sapiens

<400> 2
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30
Gly Glu Asp Ser Lys Asp Val Ala Ala Pro His Arg Gln Pro Leu Thr
        35                  40                  45
Ser Ser Glu Arg Ile Asp Lys Gln Ile Arg Tyr Ile Leu Asp Gly Ile
    50                  55                  60
Ser Ala Leu Arg Lys Glu Thr Cys Asn Lys Ser Asn Met Cys Glu Ser
65                  70                  75                  80
Ser Lys Glu Ala Leu Ala Glu Asn Asn Leu Asn Leu Pro Lys Met Ala
                85                  90                  95
Glu Lys Asp Gly Cys Phe Gln Ser Gly Phe Asn Glu Glu Thr Cys Leu
            100                 105                 110
Val Lys Ile Ile Thr Gly Leu Leu Glu Phe Glu Val Tyr Leu Glu Tyr
        115                 120                 125
Leu Gln Asn Arg Phe Glu Ser Ser Glu Glu Gln Ala Arg Ala Val Gln
    130                 135                 140
Met Ser Thr Lys Val Leu Ile Gln Phe Leu Gln Lys Lys Ala Lys Asn
145                 150                 155                 160
Leu Asp Ala Ile Thr Thr Pro Asp Pro Thr Thr Asn Ala Ser Leu Leu
                165                 170                 175
Thr Lys Leu Gln Ala Gln Asn Gln Trp Leu Gln Asp Met Thr Thr His
            180                 185                 190
Leu Ile Leu Arg Ser Phe Lys Glu Phe Leu Gln Ser Ser Leu Arg Ala
        195                 200                 205
Leu Arg Gln Met
    210

<210> 3
<211> 8704
<212> RNA
<213> Homo sapiens

<400> 3
   agttccttaa tcatttcacg gtgccttcgg acgctttttt tccacctaaa acgtttagtt      60
   tcagctcagt gatcagctac cccagctcgg cgggggagcg gaaggcttga attattccga     120
   cctgtgagcg gcccctggca ccaaaaaaaa aaaaaaaaaa aaaaaaaaaa gaaaaaaaaa     180
   ggcacaaaaa agtggaaact tttccctgtc cattccatca agtcctgaaa aatcaaaatg     240
   gatttagaga aaaattatcc gactcctcgg accagcagga caggacatgg aggagtgaat     300
   cagcttgggg gggtttttgt gaatggacgg ccactcccgg atgtagtccg ccagaggata     360
   gtggaacttg ctcatcaagg tgtcaggccc tgcgacatct ccaggcagct tcgggtcagc     420
   catggttgtg tcagcaaaat tcttggcagg tattatgaga caggaagcat caagcctggg     480
   gtaattggag gatccaaacc aaaggtcgcc acacccaaag tggtggaaaa aatcgctgaa     540
   tataaacgcc aaaatcccac catgtttgcc tgggagatca gggaccggct gctggcagag     600
   cgggtgtgtg acaatgacac cgtgcctagc gtcagttcca tcaacaggat catccggaca     660
   aaagtacagc agccacccaa ccaaccagtc ccagcttcca gtcacagcat agtgtccact     720
   ggctccgtga cgcaggtgtc ctcggtgagc acggattcgg ccggctcgtc gtactccatc     780
   agcggcatcc tgggcatcac gtcccccagc gccgacacca acaagcgcaa gagagacgaa     840
   ggtattcagg agtctccggt gccgaacggc cactcgcttc cgggcagaga cttcctccgg     900
   aagcagatgc ggggagactt gttcacacag cagcagctgg aggtgctgga ccgcgtgttt 960
   gagaggcagc actactcaga catcttcacc accacagagc ccatcaagcc cgagcagacc 1020
   acagagtatt cagccatggc ctcgctggct ggtgggctgg acgacatgaa ggccaatctg 1080
   gccagcccca cccctgctga catcgggagc agtgtgccag gcccgcagtc ctaccccatt 1140
   gtgacaggcc gtgacttggc gagcacgacc ctccccgggt accctccaca cgtccccccc 1200
   gctggacagg gcagctactc agcaccgacg ctgacaggga tggtgcctgg gagtgagttt 1260
   tccgggagtc cctacagcca ccctcagtat tcctcgtaca acgactcctg gaggttcccc 1320
   aacccggggc tgcttggctc cccctactat tatagcgctg ccgcccgagg agccgcccca 1380
   cctgcagccg ccactgccta tgaccgtcac tgacccttgg agccaggcgg gcaccaaaca 1440
   ctgatggcac ctattgaggg tgacagccac ccagccctcc tgaagatagc cagagagccc 1500
   atgagaccgt cccccagcat cccccacttg cctgaagctc ccctcttcct ctcttcctcc 1560
   agggactctg gggccctttg gtggggccgt tggacttctg gatgcttgtc tatttctaaa 1620
   agccaatcta tgagcttctc ccgatggcca ctgggtctct gcaaaccaat agactgtcct 1680
   gcaaataacc gcagccccag cccagcctgc ctgtcctcca gctgtctgac tatccatcca 1740
   tcataaccac cccagcctgg gaaggagagc ttgcttttgt tgcttcagca gcacccatgt 1800
   aaataccttc ttgcttttct gtgggcctga aggtccgact gagaagactg ctccacccat 1860
   gatgcatctc gcactcttgg tgcatcaccg gacatcttag acctatggca gagcatcctc 1920
   tctgccctgg gtgaccctgg caggtgcgct cagagctgtc ctcaagatgg aggatgctgc 1980
   ccttgggccc cagcctcctg ctcatccctc cttctttagt atctttacga ggagtctcac 2040
   tgggctggtt gtgctgcagg ctccccctga ggcccctctc caagaggagc acactttggg 2100
   gagatgtcct ggtttcctgc ctccatttct ctgggaccga tgcagtatca gcagctcttt 2160
   tccagatcaa agaactcaaa gaaaactgtc tgggagattc ctcagctact tttccgaagc 2220
   agaatgtcat ccgaggtatt gattacattg tggactttga atgtgagggc tggatgggac 2280
   gcaggagatc atctgatccc agccaaggag gggcctgagg ctctccctac tccctcagcc 2340
   cctggaacgg tgttttctga ggcatgccca ggttcaggtc acttcggaca cctgccatgg 2400
   acacttcacc caccctccag gaccccagca agtggattct gggcaagcct gttccggtga 2460
   tgtagacaat aattaacaca gaggactttc ccccacaccc agatcacaaa cagcctacag 2520
   ccagaacttc tgagcatcct ctcggggcag accctccccg tcctcgtgga gcttagcagg 2580
   cagctgggca tggaggtgct ggggctgggg cagatgccta atttcgcaca atgcatgccc 2640
   acctgttgat ctaaggggcc gcgatggtca gggccacggc caaggccacg gggaacttgg 2700
   agagggagct tggagaactc actgtgggct agggtggtca gaggaagcca gcagggaaga 2760
   tctgggggac agaggaaggc ctcctgaggg aggggcagga gagcagtgag gagctgctgt 2820
   gtgacctggg agtgattttg acatgggggt gccaggtgcc atcatctctt tacctggggc 2880
   cttaattcct tgcatagtct ctcttgtcaa gtcagaacag ccaggtagag cccttgtcca 2940
   aacctgggct gaatgacagt gatgagaggg ggcttggcct tcttaggtga caatgtcccc 3000
   catatctgta tgtcaccagg atggcagaga gccagggcag agagagactg gacttgggat 3060
   cagcaggcca ggcaggtctt gtcctggtcc tggccacatg tctttgctgt gggacctcag 3120
   acaaaaccct gcacctcttt gagccttggc tgccttggtg cagcagggtc atctgtaggg 3180
   ccaccccaca gctctttcct tcccctcctc tctccaggga gccggggctg tgagaggatc 3240
   atctggggca ggccctccac ttccaagcaa gcagatgggg gtgggcacct gaggcccaat 3300
   aatatttgga ccaagtggga aacaagaaca ctcggagggg cgggaatcag aagagcctgg 3360
   aaaaagacct agcccaactt cccttgtggg aaactgaggc ccagcttggg gaaggccagg 3420
   accatgcagg gagaaaaagc agacttcctc tggccaccgc taagtatttt gttccctaag 3480
   tcccccacag ggtggtggaa cagaagagaa aactaaggct cagcaaggtg gagtcctcaa 3540
   gcagtgactg gtgggggtgg ggctgggact cgggctcctg acccccaacc catggtgttc 3600
   cctgtcaccc tggtctatcc acatctccta ttcctgagga gagttgacag taagagcagt 3660
   gagagatggt tctgggcccc atgccctaga caatcagtct gtaagaactg ccaaggaagc 3720
   ctggtcaccc aggccaggga tggagcccag cgagctcaca gcaggcacat gcacccccgc 3780
   ccccacccag aacctgcggg gcaaagaagg gaggtagttg gggccagagt ccacctccag 3840
   gagccagggt gagctggctg cagcttccac ctgtcaggta aggtaggaga aggtatgtta 3900
   cctggcatct ctctccctgc ctccctccat ttagcaggaa gtggtggggt caggggtctt 3960
   cagcacagac ttcttgagcc tctgccccct gtcacccttc ttttggaatg atgtgtaccc 4020
   acatctttgg atcctgccct ccttgtggtt ccaggctgtt gggaggaggt cagcctcccc 4080
   tgaaccagct gcctgaggca tgcagcatcc ttcctcggca aagcccacct ggctcacagc 4140
   ggccccctct ccccatcctg ttcctcttct tgccctgtaa tgagctcccc ccataccttt 4200
   cctccctcac ctgaggcttt gctggtcctc agattggttt tgtatttgtg agacaaccac 4260
   ttgactcctg ggctgccagg cggaagcaca agcgcacatg gatgcacacg gatgttctca 4320
   cacacacacg cccgctctca ccaattcaca gcacctcgtg gtccagcgga gctgcctggg 4380
   agcttggtga ggatggctcc aaaggacaca agccgttgag tagatgccag agaattctga 4440
   atggaaaaca caagtccggg gcctcaccag catcgtggca gaaggcctgg ggcatttctc 4500
   catgggcctt cttccctgtg ttcgagctct gacttttgga aaaggacatt gtggatttta 4560
   tgaaaatttc tcataccatc agtctagctc caacctagaa aaattggatg atatatcaaa 4620
   cccaacatcc ctttcccaag gcaccttagt taaggctagc ccttccataa ctgacattgt 4680
   acggtgcttt gcaatcctca accactctgt ggagcaaaga gcatgatgcc tactttatag 4740
   acggggaaat tgacatttgg ggctctcacg gcaacataga ggcgaagtga attctcagat 4800
   ctccaggccc cattctctct ccaccgagtt gtgctcctgt catgggagtg tatggcttaa 4860
   agacactccc ccacccccat tccctagaaa tcccccagac ccacaatcag gcaagaaaga 4920
   acagggaccc agaggctggc ctgacctagg ggcctgcagg ttggcggctt tgtttcctaa 4980
   gaacattgaa acctgcggag tctttgacca aatccacaac agtgcttctg aggtttcatc 5040
   cagactcttt cccagctgtc cctgaggttc agagggtatc agagtcaatt caaggccatg 5100
   ccataatccc tgaccaggcc tggacatggg tccagccctg actccagggg tccaggtgcc 5160
   aaggtcatgt gctgtccccc acttcccttt ctttgccttc gcactttgga acaggctcca 5220
   ggcctggctg tgacagtatg caggagtgcc caagccaggg ccaccagggt gtccacagcc 5280
   cctggaaaca cagatcacaa tctcaagtcc cctgaatgaa ctgcttcctg ggtgaacggg 5340
   gtggtgtagc cttgccactc gggcagcgca ccagacagta cggtgcagca gtgcccgaga 5400
   tgcccagaag tgtgcctgcc cccctgagtg gcattcaagt atgaaaactt gtaaaatttt 5460
   ctgtcggcct aaagaaaaat gtccatgggc caaacttgac ccactgggca ccagcctgtg 5520
   agccccaatg cctgtcaatt gccccctttc tcattcacct cctgtccttt gttgcagatt 5580
   tggaggagat gggagcccag agaggtgaga gatgtgcttc agggtgtcca gcaaggcagg 5640
   ggtagaaacg ggcacgccca gagcctgcca ctctcccagg cctcatcttg ggctcttgca 5700
   agtcttgcgc tttgaagatg gagttgggtg gaaggtcaga ggcgctgggg acgggattgg 5760
   ggagctgctg ggttttcagg ggaggtcagt gctgcttggg cacctttcac atgtgcaggg 5820
   aagagactca gatgtggcca cagggcactg aggggtgaaa tccatttacc agaagtcacg 5880
   ctccagaacg actgcccagc cttggcagcc agtggctcca gccaccccct cctcatgaca 5940
   ttcagccaga attgagctcc aagccaggga agcaaatctt aaaaaccaac caagcaccct 6000
   gacacagccc tagaaacacg atgagtctga aatacagctt cccaggaggg gagtctaaga 6060
   tacagcttcc cgggagggtg gaaaccaact cctgccccac ggccaggcca ggcccaggca 6120
   ggcatgggtg gatcccacag ggctctgagc tagaccggct ccacggtggc cccactacga 6180
   ggccagttct gcagtcttgg ccttgtcacc catcgagagg ctgctctgat ggttcccagc 6240
   cacacaccag ctctcctggg gaaactattt ctttcgttct tttggcctcg gagaggtccg 6300
   aggcaagtac atttcttaaa aggtaataaa atgcattatt ggaaagttgg acagtcaggc 6360
   cacgactcct agcccacggc gtgccccacc tcccagcagc cccttcagcc ccttgcccct 6420
   gttgccccaa acctcagggt tccctcttgc atattcatgg gggaaccaca gtgctgatgt 6480
   gtacttcccc actgtcagct cggctgcacc tcgtgtggcg ccaggtccca agggcctctg 6540
   cagaggccag gctgtgagcc ccttgcctgc ctgctcccct gatgaggcaa cagcttctct 6600
   gaaatgagct gccggccagg agcaggcagg caccagcctg tctttccttt tctggtaatt 6660
   cctcagcact gaggctccgt tcctgggcac cccaggattg aagggaacct cagaatcatg 6720
   tcactgccat tctagagttt caatccaagg ggtccccttt agctcatctc caagatgggt 6780
   aaacgtagcc accattcaga aagcccagaa attcttgttc ccacatctta gacccctgag 6840
   caacacaagg agaaaatgca gctgcttacc tattaatatc tactgaggga caatcagcaa 6900
   agcctcaaag gagtcgtctc aggtagggta cttggcctgt ggcaggagag acagaggcac 6960
   aaacccaccc acccatcagc ttccggtggc tgatcggggc ccagggaggg aagcaggtga 7020
   aacagcaggg tgggggtgac ttggcagtcg tgcatctcct ccccgactct gaggcctggc 7080
   aggaggaggc cacaccagcc tcacctgccc tgacccccgc cccccaccct gtgaccctgt 7140
   ggctatggcc gctggtcgcc cttgtcccca aaatcaccat gcctgtggcc acgtccctca 7200
   tcttctccaa aagcatcatt aagaacaagt gattttggat gatggatttt tgatttacaa 7260
   acggcgcatt tcccttggag tggaacagaa aggaaaccat ttaatggcgc ccttcttttc 7320
   aagcatgaat acattttaat gaaactattt tattgtattt gaggaaatgg agagttgaac 7380
   attccaacca atcaatagcc aattaattgc tataaagcta aaaagaaaat aaataaatcc 7440
   tgagtctatt ttaaacactg caaaaagttc agagcctcag aatctggcct tcccctccat 7500
   aaggtgcacg agcatgtaaa cacacacaca cacacacaca cacacacaca cacactcact 7560
   cactccccta cacctcagac atacttgaaa ctcagaaaca gcactgagtc tccccatgcc 7620
   aattcttgcc tgctgtcttc gacttgggtc agagaaggtg agcagacccg gcagcagcct 7680
   gtcccggggc tcaggaagag gcaggcccat cccctggccc caagcaccca gcacaacaga 7740
   gggtggcggg cagtgagggc ctggcgttgc ctgggcccca cttctcagcc ccagctgctg 7800
   ggcctccaag gttgggctga ggatggagtt ttggctctgg gtttgccctg actcctgctg 7860
   gaagacgctg ccctggtttt tcaccctcta gtggccttgg acattgagta tttgtagaaa 7920
   tgcagattac attgcaaatg gaaacctttg ccaggaagac acatgcattt tgcttttaat 7980
   tctttgagac atttgatttt gtcttaggga ctgacctttc agcatcaaag aaatacatat 8040
   ctactgtatc cgccaaagtt tgtgatgcct gcatagacgc ttacttgtaa aaaaaaaaaa 8100
   atacaaaaaa atacaaaaaa accaacaaca aaaaccacaa ttgaattgcc tttgaaagtg 8160
   ggagatgatc tgtctccaac ggattgaaaa aaaaaaatgc ttcttaaaaa atgtgtatgt 8220
   tttgtattct ttttttctag tagaaaataa ctgacttgaa atattggtgg tttttttctt 8280
   agtgacgtgt gttgcttttg tgtgtaataa tatttgaatg taattacagc agtgccaatt 8340
   tgccaaagat gttggacata tttttctttt ttggggagga gggcaggcct aggggtggga 8400
   cttgggagaa aacaggggtg gggttttggt ttaatttttt ttttactttt ttttccttgt 8460
   caaacctgaa atttgtggct tccttttaag ttaaatggtt gactgcaaca cctttatttt 8520
   agattagttg gagaaacatg caataagatt ggcgtagttt caatatctgt gtgtcttttc 8580
   atgagtggct gttacttgtg aagaattgat tttatgtaac ctttatgtga gataattatt 8640
   tgtaaatatt tgccataatt ttattggttc ctaaaataaa agtaattttt taagttcaga 8700
   aaaa 8704

<210> 4
<211> 8602
<212> RNA
<213> Homo sapiens

<400> 4
   agttccttaa tcatttcacg gtgccttcgg acgctttttt tccacctaaa acgtttagtt 60
   tcagctcagt gatcagctac cccagctcgg cgggggagcg gaaggcttga attattccga 120
   cctgtgagcg gcccctggca ccaaaaaaaa aaaaaaaaaa aaaaaaaaaa gaaaaaaaaa 180
   ggcacaaaaa agtggaaact tttccctgtc cattccatca agtcctgaaa aatcaaaatg 240
   gatttagaga aaaattatcc gactcctcgg accagcagga caggacatgg aggagtgaat 300
   cagcttgggg gggtttttgt gaatggacgg ccactcccgg atgtagtccg ccagaggata 360
   gtggaacttg ctcatcaagg tgtcaggccc tgcgacatct ccaggcagct tcgggtcagc 420
   catggttgtg tcagcaaaat tcttggcagg tattatgaga caggaagcat caagcctggg 480
   gtaattggag gatccaaacc aaaggtcgcc acacccaaag tggtggaaaa aatcgctgaa 540
   tataaacgcc aaaatcccac catgtttgcc tgggagatca gggaccggct gctggcagag 600
   cgggtgtgtg acaatgacac cgtgcctagc gtcagttcca tcaacaggat catccggaca 660
   aaagtacagc agccacccaa ccaaccagtc ccagcttcca gtcacagcat agtgtccact 720
   ggctccgtga cgcaggtgtc ctcggtgagc acggattcgg ccggctcgtc gtactccatc 780
   agcggcatcc tgggcatcac gtcccccagc gccgacacca acaagcgcaa gagagacgaa 840
   ggtattcagg agtctccggt gccgaacggc cactcgcttc cgggcagaga cttcctccgg 900
   aagcagatgc ggggagactt gttcacacag cagcagctgg aggtgctgga ccgcgtgttt 960
   gagaggcagc actactcaga catcttcacc accacagagc ccatcaagcc cgagcagacc 1020
   acagagtatt cagccatggc ctcgctggct ggtgggctgg acgacatgaa ggccaatctg 1080
   gccagcccca cccctgctga catcgggagc agtgtgccag gcccgcagtc ctaccccatt 1140
   gtgacaggga gtgagttttc cgggagtccc tacagccacc ctcagtattc ctcgtacaac 1200
   gactcctgga ggttccccaa cccggggctg cttggctccc cctactatta tagcgctgcc 1260
   gcccgaggag ccgccccacc tgcagccgcc actgcctatg accgtcactg acccttggag 1320
   ccaggcgggc accaaacact gatggcacct attgagggtg acagccaccc agccctcctg 1380
   aagatagcca gagagcccat gagaccgtcc cccagcatcc cccacttgcc tgaagctccc 1440
   ctcttcctct cttcctccag ggactctggg gccctttggt ggggccgttg gacttctgga 1500
   tgcttgtcta tttctaaaag ccaatctatg agcttctccc gatggccact gggtctctgc 1560
   aaaccaatag actgtcctgc aaataaccgc agccccagcc cagcctgcct gtcctccagc 1620
   tgtctgacta tccatccatc ataaccaccc cagcctggga aggagagctt gcttttgttg 1680
   cttcagcagc acccatgtaa ataccttctt gcttttctgt gggcctgaag gtccgactga 1740
   gaagactgct ccacccatga tgcatctcgc actcttggtg catcaccgga catcttagac 1800
   ctatggcaga gcatcctctc tgccctgggt gaccctggca ggtgcgctca gagctgtcct 1860
   caagatggag gatgctgccc ttgggcccca gcctcctgct catccctcct tctttagtat 1920
   ctttacgagg agtctcactg ggctggttgt gctgcaggct ccccctgagg cccctctcca 1980
   agaggagcac actttgggga gatgtcctgg tttcctgcct ccatttctct gggaccgatg 2040
   cagtatcagc agctcttttc cagatcaaag aactcaaaga aaactgtctg ggagattcct 2100
   cagctacttt tccgaagcag aatgtcatcc gaggtattga ttacattgtg gactttgaat 2160
   gtgagggctg gatgggacgc aggagatcat ctgatcccag ccaaggaggg gcctgaggct 2220
   ctccctactc cctcagcccc tggaacggtg ttttctgagg catgcccagg ttcaggtcac 2280
   ttcggacacc tgccatggac acttcaccca ccctccagga ccccagcaag tggattctgg 2340
   gcaagcctgt tccggtgatg tagacaataa ttaacacaga ggactttccc ccacacccag 2400
   atcacaaaca gcctacagcc agaacttctg agcatcctct cgggcagacc cctccccgtc 2460
   ctcgtggagc ttagcaggca gctgggcatg gaggtgctgg ggctggggca gatgcctaat 2520
   ttcgcacaat gcatgcccac ctgttgatct aaggggccgc gatggtcagg gccacggcca 2580
   agggccacgg gaacttggag agggagcttg gagaactcac tgtgggctag ggtggtcaga 2640
   ggaagccagc agggaagatc tgggggacag aggaaggcct cctgagggag gggcaggaga 2700
   gcagtgagga gctgctgtgt gacctgggag tgattttgac atgggggtgc caggtgccat 2760
   catctcttta cctggggcct taattccttg catagtctct cttgtcaagt cagaacagcc 2820
   aggtagagcc cttgtccaaa cctgggctga atgacagtga tgagaggggg cttggccttc 2880
   ttaggtgaca atgtccccca tatctgtatg tcaccaggat ggcagagagc cagggcagag 2940
   agagactgga cttgggatca gcaggccagg caggtcttgt cctggtcctg gccacatgtc 3000
   tttgctgtgg gacctcagac aaaaccctgc acctctttga gccttggctg ccttggtgca 3060
   gcagggtcat ctgtagggcc accccacagc tctttccttc ccctcctctc tccagggagc 3120
   cggggctgtg agaggatcat ctggggcagg ccctccactt ccaagcaagc agatgggggt 3180
   gggcacctga ggcccaataa tatttggacc aagtgggaaa caagaacact cggaggggcg 3240
   ggaatcagaa gagcctggaa aaagacctag cccaacttcc cttgtgggaa actgaggccc 3300
   agcttgggga aggccaggac catgcaggga gaaaaagcag acttcctctg gccaccgcta 3360
   agtattttgt tccctaagtc ccccacaggg tggtggaaca gaagagaaaa ctaaggctca 3420
   gcaaggtgga gtcctcaagc agtgactggt gggggtgggg ctgggactcg ggctcctgac 3480
   ccccaaccca tggtgttccc tgtcaccctg gtctatccac atctcctatt cctgaggaga 3540
   gttgacagta agagcagtga gagatggttc tgggccccat gccctagaca atcagtctgt 3600
   aagaactgcc aaggaagcct ggtcacccag gccagggatg gagcccagcg agctcacagc 3660
   aggcacatgc acccccgccc ccacccagaa cctgcggggc aaagaaggga ggtagttggg 3720
   gccagagtcc acctccagga gccagggtga gctggctgca gcttccacct gtcaggtaag 3780
   gtaggagaag gtatgttacc tggcatctct ctccctgcct ccctccattt agcaggaagt 3840
   ggtggggtca ggggtcttca gcacagactt cttgagcctc tgccccctgt cacccttctt 3900
   ttggaatgat gtgtacccac atctttggat cctgccctcc ttgtggttcc aggctgttgg 3960
   gaggaggtca gcctcccctg aaccagctgc ctgaggcatg cagcatcctt cctcggcaaa 4020
   gcccacctgg ctcacagcgg ccccctctcc ccatcctgtt cctcttcttg ccctgtaatg 4080
   agctcccccc atacctttcc tccctcacct gaggctttgc tggtcctcag attggttttg 4140
   tatttgtgag acaaccactt gactcctggg ctgccaggcg gaagcacaag cgcacatgga 4200
   tgcacacgga tgttctcaca cacacacgcc cgctctcacc aattcacagc acctcgtggt 4260
   ccagcggagc tgcctgggag cttggtgagg atggctccaa aggacacaag ccgttgagta 4320
   gatgccagag aattctgaat ggaaaacaca agtccggggc ctcaccagca tcgtggcaga 4380
   aggcctgggg catttctcca tgggccttct tccctgtgtt cgagctctga cttttggaaa 4440
   aggacattgt ggattttatg aaaatttctc ataccatcag tctagctcca acctagaaaa 4500
   attggatgat atatcaaacc caacatccct ttcccaaggc accttagtta aggctagccc 4560
   ttccataact gacattgtac ggtgctttgc aatcctcaac cactctgtgg agcaaagagc 4620
   atgatgccta ctttatagac ggggaaattg acatttgggg ctctcacggc aacatagagg 4680
   cgaagtgaat tctcagatct ccaggcccca ttctctctcc accgagttgt gctcctgtca 4740
   tgggagtgta tggcttaaag acactccccc acccccattc cctagaaatc ccccagaccc 4800
   acaatcaggc aagaaagaac agggacccag aggctggcct gacctagggg cctgcaggtt 4860
   ggcggctttg tttcctaaga acattgaaac ctgcggagtc tttgaccaaa tccacaacag 4920
   tgcttctgag gtttcatcca gactctttcc cagctgtccc tgaggttcag agggtatcag 4980
   agtcaattca aggccatgcc ataatccctg accaggcctg gacatgggtc cagccctgac 5040
   tccaggggtc caggtgccaa ggtcatgtgc tgtcccccac ttccctttct ttgccttcgc 5100
   actttggaac aggctccagg cctggctgtg acagtatgca ggagtgccca agccagggcc 5160
   accagggtgt ccacagcccc tggaaacaca gatcacaatc tcaagtcccc tgaatgaact 5220
   gcttcctggg tgaacggggt ggtgtagcct tgccactcgg gcagcgcacc agacagtacg 5280
   gtgcagcagt gcccgagatg cccagaagtg tgcctgcccc cctgagtggc attcaagtat 5340
   gaaaacttgt aaaatttctt gtcggcctaa agaaaaatgt ccatgggcca aacttgaccc 5400
   actgggcacc agcctgtgag ccccaatgcc tgtcaattgc cccctttctc attcacctcc 5460
   tgtcctttgt tgcagatttg gaggagatgg gagcccagag aggtgagaga tgtgcttcag 5520
   ggtgtccagc aaggcagggg tagaaacggg cacgcccaga gcctgccact ctcccaggcc 5580
   tcatcttggg ctcttgcaag tcttgcgctt tgaagatgga gttgggtgga aggtcagagg 5640
   cgctggggac gggattgggg agctgctggg ttttcagggg aggtcagtgc tgcttgggca 5700
   cctttcacat gtgcagggaa gagactcaga tgtggccaca gggcactgag gggtgaaatc 5760
   catttaccag aagtcacgct ccagaacgac tgcccagcct tggcagccag tggctccagc 5820
   caccccctcc tcatgacatt cagccagaat tgagctccaa gccagggaag caaatcttaa 5880
   aaaccaacca agcaccctga cacagcccta gaaacacgat gagtctgaaa tacagcttcc 5940
   caggagggga gtctaagata cagcttcccg ggagggtgga aaccaactcc tgccccacgg 6000
   ccaggccagg cccaggcagg catgggtgga tcccacaggg ctctgagcta gaccggctcc 6060
   acggtggccc cactacgagg ccagttctgc agtcttggcc ttgtcaccca tcgagaggct 6120
   gctctgatgg ttcccagcca cacaccagct ctcctgggga aactatttct ttcgttcttt 6180
   tggcctcgga gaggtccgag gcaagtacat ttcttaaaag gtaataaaat gcattattgg 6240
   aaagttggac agtcaggcca cgactcctag cccacggcgt gccccacctc ccagcagccc 6300
   cttcagcccc ttgcccctgt tgccccaaac ctcagggttc cctcttgcat attcatgggg 6360
   gaaccacagt gctgatgtgt acttccccac tgtcagctcg gctgcacctc gtgtggcgcc 6420
   aggtcccaag ggcctctgca gaggccaggc tgtgagcccc ttgcctgcct gctcccctga 6480
   tgaggcaaca gcttctctga aatgagctgc cggccaggag caggcaggca ccagcctgtc 6540
   tttccttttc tggtaattcc tcagcactga ggctccgttc ctgggcaccc caggattgaa 6600
   gggaacctca gaatcatgtc actgccattc tagagtttca atccaagggg tcccctttag 6660
   ctcatctcca agatgggtaa acgtagccac cattcagaaa gcccagaaat tcttgttccc 6720
   acatcttaga cccctgagca acacaaggag aaaatgcagc tgcttaccta ttaatatcta 6780
   ctgaggacaa atcagcaaag cctcaaagga gtcgtctcag gtagggtact tggcctgtgg 6840
   caggagagac agaggcacaa acccacccac ccatcagctt ccggtggctg atcggggccc 6900
   agggagggaa gcaggtgaaa cagcaggtgg ggggtgactt ggcagtcgtg catctcctcc 6960
   ccgactctga ggcctggcag gaggaggcca caccagcctc acctgccctg acccccgccc 7020
   cccaccctgt gaccctgtgg ctatggccgc tggtcgccct tgtccccaaa atcaccatgc 7080
   ctgtggccac gtccctcatc ttctccaaaa gcatcattaa gaacaagtga ttttggatga 7140
   tggatttttg atttacaaac ggcgcatttc ccttggagtg gaacagaaag gaaaccattt 7200
   aatggcgccc ttcttttcaa gcatgaatac attttaatga aactatttta ttgtatttga 7260
   ggaaatggag agttgaacat tccaaccaat caatagccaa ttaattgcta taaagctaaa 7320
   aagaaaataa ataaatcctg agtctatttt aaacactgca aaaagttcag agcctcagaa 7380
   tctggccttc ccctccataa ggtgcacgag catgtaaaca cacacacaca cacacacaca 7440
   cacacacaca cactcactca ctcccctaca cctcagacat acttgaaact cagaaacagc 7500
   actgagtctc cccatgccaa ttcttgcctg ctgtcttcga cttgggtcag agaaggtgag 7560
   cagacccggc agcagcctgt cccggggctc aggaagaggc aggcccatcc cctggcccca 7620
   agcacccagc acaacagagg gtggcgggca gtgagggcct ggcgttgcct gggccccact 7680
   tctcagcccc agctgctggg cctccaaggt tgggctgagg atggagtttt ggctctgggt 7740
   ttgccctgac tcctgctgga agacgctgcc ctggtttttc accctctagt ggccttggac 7800
   attgagtatt tgtagaaatg cagattacat tgcaaatgga aacctttgcc aggaagacac 7860
   atgcattttg cttttaattc tttgagacat ttgattttgt cttagggact gacctttcag 7920
   catcaaagaa atacatatct actgtatccg ccaaagtttg tgatgcctgc atagacgctt 7980
   acttgtaaaa aaaaaaaaat acaaaaaaat acaaaaaaac caacaacaaa aaccacaatt 8040
   gaattgcctt tgaaagtggg agatgatctg tctccaacgg attgaaaaaa aaaaatgctt 8100
   cttaaaaaat gtgtatgttt tgtattcttt ttttctagta gaaaataact gacttgaaat 8160
   attggtggtt tttttcttag tgacgtgtgt tgcttttgtg tgtaataata tttgaatgta 8220
   attacagcag tgccaatttg ccaaagatgt tggacatatt tttctttttt ggggaggagg 8280
   gcagggctag gggtgggact tgggagaaaa caggggtggg gttttggttt aatttttttt 8340
   ttactttttt ttccttgtca aacctgaaat ttgtggcttc cttttaagtt aaatggttga 8400
   ctgcaacacc tttattttag attagttgga gaaacatgca ataagattgg cgtagtttca 8460
   atatctgtgt gtcttttcat gagtggctgt tacttgtgaa gaattgattt tatgtaacct 8520
   ttatgtgaga taattatttg taaatatttg ccataatttt attggttcct aaaataaaag 8580
   taatttttta agttcagaaa aa 8602

<210> 5
<211> 8617
<212> RNA
<213> Homo sapiens

<400> 5

agttccttaa tcatttcacg gtgccttcgg acgctttttt tccacctaaa acgtttagtt 60

tcagctcagt gatcagctac cccagctcgg cgggggagcg gaaggcttga attattccga 120

cctgtgagcg gcccctggca ccaaaaaaaa aaaaaaaaaa aaaaaaaaaa gaaaaaaaaa 180

ggcacaaaaa agtggaaact tttccctgtc cattccatca agtcctgaaa aatcaaaatg 240

gatttagaga aaaattatcc gactcctcgg accagcagga caggacatgg aggagtgaat 300

cagcttgggg gggtttttgt gaatggacgg ccactcccgg atgtagtccg ccagaggata 360

gtggaacttg ctcatcaagg tgtcaggccc tgcgacatct ccaggcagct tcgggtcagc 420

catggttgtg tcagcaaaat tcttggcagg tattatgaga caggaagcat caagcctggg 480

gtaattggag gatccaaacc aaaggtcgcc acacccaaag tggtggaaaa aatcgctgaa 540

tataaacgcc aaaatcccac catgtttgcc tgggagatca gggaccggct gctggcagag 600

cgggtgtgtg acaatgacac cgtgcctagc gtcagttcca tcaacaggat catccggaca 660

aaagtacagc agccacccaa ccaaccagtc ccagcttcca gtcacagcat agtgtccact 720

ggctccgtga cgcaggtgtc ctcggtgagc acggattcgg ccggctcgtc gtactccatc 780

agcggcatcc tgggcatcac gtcccccagc gccgacacca acaagcgcaa gagagacgaa 840

ggtattcagg agtctccggt gccgaacggc cactcgcttc cgggcagaga cttcctccgg 900

aagcagatgc ggggagactt gttcacacag cagcagctgg aggtgctgga ccgcgtgttt 960

gagaggcagc actactcaga catcttcacc accacagagc ccatcaagcc cgagcagacc 1020

acagagtatt cagccatggc ctcgctggct ggtgggctgg acgacatgaa ggccaatctg 1080

gccagcccca cccctgctga catcgggagc agtgtgccag gcccgcagtc ctaccccatt 1140

gtgacaggcc gtgacttggc gagcacgacc ctccccgggt accctccaca cgtccccccc 1200

gctggacagg gcagctactc agcaccgacg ctgacaggga tggtgcctgg ctccccctac 1260

tattatagcg ctgccgcccg aggagccgcc ccacctgcag ccgccactgc ctatgaccgt 1320

cactgaccct tggagccagg cgggcaccaa acactgatgg cacctattga gggtgacagc 1380

cacccagccc tcctgaagat agccagagag cccatgagac cgtcccccag catcccccac 1440

ttgcctgaag ctcccctctt cctctcttcc tccagggact ctggggccct ttggtggggc 1500

cgttggactt ctggatgctt gtctatttct aaaagccaat ctatgagctt ctcccgatgg 1560

ccactgggtc tctgcaaacc aatagactgt cctgcaaata accgcagccc cagcccagcc 1620

tgcctgtcct ccagctgtct gactatccat ccatcataac caccccagcc tgggaaggag 1680

agcttgcttt tgttgcttca gcagcaccca tgtaaatacc ttcttgcttt tctgtgggcc 1740

tgaaggtccg actgagaaga ctgctccacc catgatgcat ctcgcactct tggtgcatca 1800

ccggacatct tagacctatg gcagagcatc ctctctgccc tgggtgaccc tggcaggtgc 1860

gctcagagct gtcctcaaga tggaggatgc tgcccttggg ccccagcctc ctgctcatcc 1920

ctccttcttt agtatcttta cgaggagtct cactgggctg gttgtgctgc aggctccccc 1980

tgaggcccct ctccaagagg agcacacttt ggggagatgt cctggtttcc tgcctccatt 2040

tctctgggac cgatgcagta tcagcagctc ttttccagat caaagaactc aaagaaaact 2100

gtctgggaga ttcctcagct acttttccga agcagaatgt catccgaggt attgattaca 2160

ttgtggactt tgaatgtgag ggctggatgg gacgcaggag atcatctgat cccagccaag 2220

gaggggcctg aggctctccc tactccctca gcccctggaa cggtgttttc tgaggcatgc 2280

ccaggttcag gtcacttcgg acacctgcca tggacacttc acccaccctc caggacccca 2340

gcaagtggat tctgggcaag cctgttccgg tgatgtagac aataattaac acagaggact 2400

ttcccccaca cccagatcac aaacagccta cagccagaac ttctgagcat cctctcgggg 2460

cagaccctcc ccgtcctcgt ggagcttagc aggcagctgg gcatggaggt gctggggctg 2520

gggcagatgc ctaatttcgc acaatgcatg cccacctgtt gatctaaggg gccgcgatgg 2580

tcagggccac ggccaagggc cacgggaact tggagaggga gcttggagaa ctcactgtgg 2640

gctagggtgg tcagaggaag ccagcaggga agatctgggg gacagaggaa ggcctcctga 2700

gggaggggca ggagagcagt gaggagctgc tgtgtgacct gggagtgatt ttgacatggg 2760

ggtgccaggt gccatcatct ctttacctgg ggccttaatt ccttgcatag tctctcttgt 2820

caagtcagaa cagccaggta gagcccttgt ccaaacctgg gctgaatgac agtgatgaga 2880

gggggcttgg ccttcttagg tgacaatgtc ccccatatct gtatgtcacc aggatggcag 2940

agagccaggg cagagagaga ctggacttgg gatcagcagg ccaggcaggt cttgtcctgg 3000

tcctggccac atgtctttgc tgtgggacct cagacaaaac cctgcacctc tttgagcctt 3060

ggctgccttg gtgcagcagg gtcatctgta gggccacccc acagctcttt ccttcccctc 3120

ctctctccag ggagccgggg ctgtgagagg atcatctggg gcaggccctc cacttccaag 3180

caagcagatg ggggtgggca cctgaggccc aataatattt ggaccaagtg ggaaacaaga 3240

acactcggag gggcgggaat cagaagagcc tggaaaaaga cctagcccaa cttcccttgt 3300

gggaaactga ggcccagctt ggggaaggcc aggaccatgc agggagaaaa agcagacttc 3360

ctctggccac cgctaagtat tttgttccct aagtccccca cagggtggtg gaacagaaga 3420

gaaaactaag gctcagcaag gtggagtcct caagcagtga ctggtggggg tggggctggg 3480

actcgggctc ctgaccccca acccatggtg ttccctgtca ccctggtcta tccacatctc 3540

ctattcctga ggagagttga cagtaagagc agtgagagat ggttctgggc cccatgccct 3600

agacaatcag tctgtaagaa ctgccaagga agcctggtca cccaggccag ggatggagcc 3660

cagcgagctc acagcaggca catgcacccc cgcccccacc cagaacctgc ggggcaaaga 3720

agggaggtag ttggggccag agtccacctc caggagccag ggtgagctgg ctgcagcttc 3780

cacctgtcag gtaaggtagg agaaggtatg ttacctggca tctctctccc tgcctccctc 3840

catttagcag gaagtggtgg ggtcaggggt cttcagcaca gacttcttga gcctctgccc 3900

cctgtcaccc ttcttttgga atgatgtgta cccacatctt tggatcctgc cctccttgtg 3960

gttccaggct gttgggagga ggtcagcctc ccctgaacca gctgcctgag gcatgcagca 4020

tccttcctcg gcaaagccca cctggctcac agcggccccc tctccccatc ctgttcctct 4080

tcttgccctg taatgagctc cccccatacc tttcctccct cacctgaggc tttgctggtc 4140

ctcagattgg ttttgtattt gtgagacaac cacttgactc ctgggctgcc aggcggaagc 4200

acaagcgcac atggatgcac acggatgttc tcacacacac acgcccgctc tcaccaattc 4260

acagcacctc gtggtccagc ggagctgcct gggagcttgg tgaggatggc tccaaaggac 4320

acaagccgtt gagtagatgc cagagaattc tgaatggaaa acacaagtcc ggggcctcac 4380

cagcatcgtg gcagaaggcc tggggcattt ctccatgggc cttcttccct gtgttcgagc 4440

tctgactttt ggaaaaggac attgtggatt ttatgaaaat ttctcatacc atcagtctag 4500

ctccaaccta gaaaaattgg atgatatatc aaacccaaca tccctttccc aaggcacctt 4560

agttaaggct agcccttcca taactgacat tgtacggtgc tttgcaatcc tcaaccactc 4620

tgtggagcaa agagcatgat gcctacttta tagacgggga aattgacatt tggggctctc 4680

acggcaacat agaggcgaag tgaattctca gatctccagg ccccattctc tctccaccga 4740

gttgtgctcc tgtcatggga gtgtatggct taaagacact cccccacccc cattccctag 4800

aaatccccca gacccacaat caggcaagaa agaacaggga cccagaggct ggcctgacct 4860

aggggcctgc aggttggcgg ctttgtttcc taagaacatt gaaacctgcg gagtctttga 4920

ccaaatccac aacagtgctt ctgaggtttc atccagactc tttcccagct gtccctgagg 4980

ttcagagggt atcagagtca attcaaggcc atgccataat ccctgaccag gcctggacat 5040

gggtccagcc ctgactccag gggtccaggt gccaaggtca tgtgctgtcc cccacttccc 5100

tttctttgcc ttcgcacttt ggaacaggct ccaggcctgg ctgtgacagt atgcaggagt 5160

gcccaagcca gggccaccag ggtgtccaca gcccctggaa acacagatca caatctcaag 5220

tcccctgaat gaactgcttc ctgggtgaac ggggtggtgt agccttgcca ctcgggcagc 5280

gcaccagaca gtacggtgca gcagtgcccg agatgcccag aagtgtgcct gcccccctga 5340

gtggcattca agtatgaaaa cttgtaaaat tttctgtcgg cctaaagaaa aatgtccatg 5400

ggccaaactt gacccactgg gcaccagcct gtgagcccca atgcctgtca attgccccct 5460

ttctcattca cctcctgtcc tttgttgcag atttggagga gatgggagcc cagagaggtg 5520

agagatgtgc ttcagggtgt ccagcaaggc aggggtagaa acgggcacgc ccagagcctg 5580

ccactctccc aggcctcatc ttgggctctt gcaagtcttg cgctttgaag atggagttgg 5640

gtggaaggtc agaggcgctg gggacgggat tggggagctg ctgggttttc aggggaggtc 5700

agtgctgctt gggcaccttt cacatgtgca gggaagagac tcagatgtgg ccacagggca 5760

ctgaggggtg aaatccattt accagaagtc acgctccaga acgactgccc agccttggca 5820

gccagtggct ccagccaccc cctcctcatg acattcagcc agaattgagc tccaagccag 5880

ggaagcaaat cttaaaaacc aaccaagcac cctgacacag ccctagaaac acgatgagtc 5940

tgaaatacag cttcccagga ggggagtcta agatacagct tcccgggagg gtggaaacca 6000

actcctgccc cacggccagg ccaggcccag gcaggcatgg gtggatccca cagggctctg 6060

agctagaccg gctccacggt ggccccacta cgaggccagt tctgcagtct tggccttgtc 6120

acccatcgag aggctgctct gatggttccc agccacacac cagctctcct ggggaaacta 6180

tttctttcgt tcttttggcc tcggagaggt ccgaggcaag tacatttctt aaaaggtaat 6240

aaaatgcatt attggaaagt tggacagtca ggccacgact cctagcccac ggcgtgcccc 6300

acctcccagc agccccttca gccccttgcc cctgttgccc caaacctcag ggttccctct 6360

tgcatattca tgggggaacc acagtgctga tgtgtacttc cccactgtca gctcggctgc 6420

acctcgtgtg gcgccaggtc ccaagggcct ctgcagaggc caggctgtga gccccttgcc 6480

tgcctgctcc cctgatgagg caacagcttc tctgaaatga gctgccggcc aggagcaggc 6540

aggcaccagc ctgtctttcc ttttctggta attcctcagc actgaggctc cgttcctggg 6600

caccccagga ttgaagggaa cctcagaatc atgtcactgc cattctagag tttcaatcca 6660

aggggtcccc tttagctcat ctccaagatg ggtaaacgta gccaccattc agaaagccca 6720

gaaattcttg ttcccacatc ttagacccct gagcaacaca aggagaaaat gcagctgctt 6780

acctattaat atctactgag ggacaatcag caaagcctca aaggagtcgt ctcaggtagg 6840

gtacttggcc tgtggcagga gagacagagg cacaaaccca cccacccatc agcttccggt 6900

ggctgatcgg ggcccaggga gggaagcagg tgaaacagca gggtgggggt gacttggcag 6960

tcgtgcatct cctccccgac tctgaggcct ggcaggagga ggccacacca gcctcacctg 7020

ccctgacccc cgccccccac cctgtgaccc tgtggctatg gccgctggtc gcccttgtcc 7080

ccaaaatcac catgcctgtg gccacgtccc tcatcttctc caaaagcatc attaagaaca 7140

agtgattttg gatgatggat ttttgattta caaacggcgc atttcccttg gagtggaaca 7200

gaaaggaaac catttaatgg cgcccttctt ttcaagcatg aatacatttt aatgaaacta 7260

ttttattgta tttgaggaaa tggagagttg aacattccaa ccaatcaata gccaattaat 7320

tgctataaag ctaaaaagaa aataaataaa tcctgagtct attttaaaca ctgcaaaaag 7380

ttcagagcct cagaatctgg ccttcccctc cataaggtgc acgagcatgt aaacacacac 7440

acacacacac acacacacac acacacactc actcactccc ctacacctca gacatacttg 7500

aaactcagaa acagcactga gtctccccat gccaattctt gcctgctgtc ttcgacttgg 7560

gtcagagaag gtgagcagac ccggcagcag cctgtcccgg ggctcaggaa gaggcaggcc 7620

catcccctgg ccccaagcac ccagcacaac agagggtggc gggcagtgag ggcctggcgt 7680

tgcctgggcc ccacttctca gccccagctg ctgggcctcc aaggttgggc tgaggatgga 7740

gttttggctc tgggtttgcc ctgactcctg ctggaagacg ctgccctggt ttttcaccct 7800

ctagtggcct tggacattga gtatttgtag aaatgcagat tacattgcaa atggaaacct 7860

ttgccaggaa gacacatgca ttttgctttt aattctttga gacatttgat tttgtcttag 7920

ggactgacct ttcagcatca aagaaataca tatctactgt atccgccaaa gtttgtgatg 7980

cctgcataga cgcttacttg taaaaaaaaa aaaatacaaa aaaatacaaa aaaaccaaca 8040

acaaaaacca caattgaatt gcctttgaaa gtgggagatg atctgtctcc aacggattga 8100

aaaaaaaaaa tgcttcttaa aaaatgtgta tgttttgtat tctttttttc tagtagaaaa 8160

taactgactt gaaatattgg tggttttttt cttagtgacg tgtgttgctt ttgtgtgtaa 8220

taatatttga atgtaattac agcagtgcca atttgccaaa gatgttggac atatttttct 8280

tttttgggga ggagggcagg gctaggggtg ggacttggga gaaaacaggg gtggggtttt 8340

ggtttaattt tttttttact tttttttcct tgtcaaacct gaaatttgtg gcttcctttt 8400

aagttaaatg gttgactgca acacctttat tttagattag ttggagaaac atgcaataag 8460

attggcgtag tttcaatatc tgtgtgtctt ttcatgagtg gctgttactt gtgaagaatt 8520

gattttatgt aacctttatg tgagataatt atttgtaaat atttgccata attttattgg 8580

ttcctaaaat aaaagtaatt ttttaagttc agaaaaa 8617

<210> 6
<211> 8472
<212> RNA
<213> Homo sapiens

<400> 6
agttccttaa tcatttcacg gtgccttcgg acgctttttt tccacctaaa acgtttagtt 60

tcagctcagt gatcagctac cccagctcgg cgggggagcg gaaggcttga attattccga 120

cctgtgagcg gcccctggca ccaaaaaaaa aaaaaaaaaa aaaaaaaaaa gaaaaaaaaa 180

ggcacaaaaa agtggaaact tttccctgtc cattccatca agtcctgaaa aatcaaaatg 240

gatttagaga aaaattatcc gactcctcgg accagcagga caggacatgg aggagtgaat 300

cagcttgggg gggtttttgt gaatggacgg ccactcccgg atgtagtccg ccagaggata 360

gtggaacttg ctcatcaagg tgtcaggccc tgcgacatct ccaggcagct tcgggtcagc 420

catggttgtg tcagcaaaat tcttggcagg tattatgaga caggaagcat caagcctggg 480

gtaattggag gatccaaacc aaaggtcgcc acacccaaag tggtggaaaa aatcgctgaa 540

tataaacgcc aaaatcccac catgtttgcc tgggagatca gggaccggct gctggcagag 600

cgggtgtgtg acaatgacac cgtgcctagc gtcagttcca tcaacaggat catccggaca 660

aaagtacagc agccacccaa ccaaccagtc ccagcttcca gtcacagcat agtgtccact 720

ggctccgtga cgcaggtgtc ctcggtgagc acggattcgg ccggctcgtc gtactccatc 780

agcggcatcc tgggcatcac gtcccccagc gccgacacca acaagcgcaa gagagacgaa 840

ggtattcagg agtctccggt gccgaacggc cactcgcttc cgggcagaga cttcctccgg 900

aagcagatgc ggggagactt gttcacacag cagcagctgg aggtgctgga ccgcgtgttt 960

gagaggcagc actactcaga catcttcacc accacagagc ccatcaagcc cgagcaggga 1020

gtgagttttc cgggagtccc tacagccacc ctcagtattc ctcgtacaac gactcctgga 1080

ggttccccaa cccggggctg cttggctccc cctactatta tagcgctgcc gcccgaggag 1140

ccgccccacc tgcagccgcc actgcctatg accgtcactg acccttggag ccaggcgggc 1200

accaaacact gatggcacct attgagggtg acagccaccc agccctcctg aagatagcca 1260

gagagcccat gagaccgtcc cccagcatcc cccacttgcc tgaagctccc ctcttcctct 1320

cttcctccag ggactctggg gccctttggt ggggccgttg gacttctgga tgcttgtcta 1380

tttctaaaag ccaatctatg agcttctccc gatggccact gggtctctgc aaaccaatag 1440

actgtcctgc aaataaccgc agccccagcc cagcctgcct gtcctccagc tgtctgacta 1500

tccatccatc ataaccaccc cagcctggga aggagagctt gcttttgttg cttcagcagc 1560

acccatgtaa ataccttctt gcttttctgt gggcctgaag gtccgactga gaagactgct 1620

ccacccatga tgcatctcgc actcttggtg catcaccgga catcttagac ctatggcaga 1680

gcatcctctc tgccctgggt gaccctggca ggtgcgctca gagctgtcct caagatggag 1740

gatgctgccc ttgggcccca gcctcctgct catccctcct tctttagtat ctttacgagg 1800

agtctcactg ggctggttgt gctgcaggct ccccctgagg cccctctcca agaggagcac 1860

actttgggga gatgtcctgg tttcctgcct ccatttctct gggaccgatg cagtatcagc 1920

agctcttttc cagatcaaag aactcaaaga aaactgtctg ggagattcct cagctacttt 1980

tccgaagcag aatgtcatcc gaggtattga ttacattgtg gactttgaat gtgagggctg 2040

gatgggacgc aggagatcat ctgatcccag ccaaggaggg gcctgaggct ctccctactc 2100

cctcagcccc tggaacggtg ttttctgagg catgcccagg ttcaggtcac ttcggacacc 2160

tgccatggac acttcaccca ccctccagga ccccagcaag tggattctgg gcaagcctgt 2220

tccggtgatg tagacaataa ttaacacaga ggactttccc ccacacccag atcacaaaca 2280

gcctacagcc agaacttctg agcatcctct cggggcagac cctccccgtc ctcgtggagc 2340

ttagcaggca gctgggcatg gaggtgctgg ggctggggca gatgcctaat ttcgcacaat 2400

gcatgcccac ctgttgatct aaggggccgc gatggtcagg gccacggcca agggccacgg 2460

gaacttggag agggagcttg gagaactcac tgtgggctag ggtggtcaga ggaagccagc 2520

agggaagatc tgggggacag aggaaggcct cctgagggag gggcaggaga gcagtgagga 2580

gctgctgtgt gacctgggag tgattttgac atgggggtgc caggtgccat catctcttta 2640

cctggggcct taattccttg catagtctct cttgtcaagt cagaacagcc aggtagagcc 2700

cttgtccaaa cctgggctga atgacagtga tgagaggggg cttggccttc ttaggtgaca 2760

atgtccccca tatctgtatg tcaccaggat ggcagagagc cagggcagag agagactgga 2820

cttgggatca gcaggccagg caggtcttgt cctggtcctg gccacatgtc tttgctgtgg 2880

gacctcagac aaaaccctgc acctctttga gccttggctg ccttggtgca gcagggtcat 2940

ctgtagggcc accccacagc tctttccttc ccctcctctc tccagggagc cggggctgtg 3000

agaggatcat ctggggcagg ccctccactt ccaagcaagc agatgggggt gggcacctga 3060

ggcccaataa tatttggacc aagtgggaaa caagaacact cggaggggcg ggaatcagaa 3120

gagcctggaa aaagacctag cccaacttcc cttgtgggaa actgaggccc agcttgggga 3180

aggccaggac catgcaggga gaaaaagcag acttcctctg gccaccgcta agtattttgt 3240

tccctaagtc ccccacaggg tggtggaaca gaagagaaaa ctaaggctca gcaaggtgga 3300

gtcctcaagc agtgactggt gggggtgggg ctgggactcg ggctcctgac ccccaaccca 3360

tggtgttccc tgtcaccctg gtctatccac atctcctatt cctgaggaga gttgacagta 3420

agagcagtga gagatggttc tgggccccat gccctagaca atcagtctgt aagaactgcc 3480

aaggaagcct ggtcacccag gccagggatg gagcccagcg agctcacagc aggcacatgc 3540

acccccgccc ccacccagaa cctgcggggc aaagaaggga ggtagttggg gccagagtcc 3600

acctccagga gccagggtga gctggctgca gcttccacct gtcaggtaag gtaggagaag 3660

gtatgttacc tggcatctct ctccctgcct ccctccattt agcaggaagt ggtggggtca 3720

ggggtcttca gcacagactt cttgagcctc tgccccctgt cacccttctt ttggaatgat 3780

gtgtacccac atctttggat cctgccctcc ttgtggttcc aggctgttgg gaggaggtca 3840

gcctcccctg aaccagctgc ctgaggcatg cagcatcctt cctcggcaaa gcccacctgg 3900

ctcacagcgg ccccctctcc ccatcctgtt cctcttcttg ccctgtaatg agctcccccc 3960

atacctttcc tccctcacct gaggctttgc tggtcctcag attggttttg tatttgtgag 4020

acaaccactt gactcctggg ctgccaggcg gaagcacaag cgcacatgga tgcacacgga 4080

tgttctcaca cacacacgcc cgctctcacc aattcacagc acctcgtggt ccagcggagc 4140

tgcctgggag cttggtgagg atggctccaa aggacacaag ccgttgagta gatgccagag 4200

aattctgaat ggaaaacaca agtccggggc ctcaccagca tcgtggcaga aggcctgggg 4260

catttctcca tgggccttct tccctgtgtt cgagctctga cttttggaaa aggacattgt 4320

ggattttatg aaaatttctc ataccatcag tctagctcca acctagaaaa attggatgat 4380

atatcaaacc caacatccct ttcccaaggc accttagtta aggctagccc ttccataact 4440

gacattgtac ggtgctttgc aatcctcaac cactctgtgg agcaaagagc atgatgccta 4500

ctttatagac ggggaaattg acatttgggg ctctcacggc aacatagagg cgaagtgaat 4560

tctcagatct ccaggcccca ttctctctcc accgagttgt gctcctgtca tgggagtgta 4620

tggcttaaag acactccccc acccccattc cctagaaatc ccccagaccc acaatcaggc 4680

aagaaagaac agggacccag aggctggcct gacctagggg cctgcaggtt ggcggctttg 4740

tttcctaaga acattgaaac ctgcggagtc tttgaccaaa tccacaacag tgcttctgag 4800

gtttcatcca gactctttcc cagctgtccc tgaggttcag agggtatcag agtcaattca 4860

aggccatgcc ataatccctg accaggcctg gacatgggtc cagccctgac tccaggggtc 4920

caggtgccaa ggtcatgtgc tgtcccccac ttccctttct ttgccttcgc actttggaac 4980

aggctccagg cctggctgtg acagtatgca ggagtgccca agccagggcc accagggtgt 5040

ccacagcccc tggaaacaca gatcacaatc tcaagtcccc tgaatgaact gcttcctggg 5100

tgaacggggt ggtgtagcct tgccactcgg gcagcgcacc agacagtacg gtgcagcagt 5160

gcccgagatg cccagaagtg tgcctgcccc cctgagtggc attcaagtat gaaaacttgt 5220

aaaattttct gtcggcctaa agaaaaatgt ccatgggcca aacttgaccc actgggcacc 5280

agcctgtgag ccccaatgcc tgtcaattgc cccctttctc attcacctcc tgtcctttgt 5340

tgcagatttg gaggagatgg gagcccagag aggtgagaga tgtgcttcag ggtgtccagc 5400

aaggcagggg tagaaacggg cacgcccaga gcctgccact ctcccaggcc tcatcttggg 5460

ctcttgcaag tcttgcgctt tgaagatgga gttgggtgga aggtcagagg cgctggggac 5520

gggattgggg agctgctggg ttttcagggg aggtcagtgc tgcttgggca cctttcacat 5580

gtgcagggaa gagactcaga tgtggccaca gggcactgag gggtgaaatc catttaccag 5640

aagtcacgct ccagaacgac tgcccagcct tggcagccag tggctccagc caccccctcc 5700

tcatgacatt cagccagaat tgagctccaa gccagggaag caaatcttaa aaaccaacca 5760

agcaccctga cacagcccta gaaacacgat gagtctgaaa tacagcttcc caggagggga 5820

gtctaagata cagcttcccg ggagggtgga aaccaactcc tgccccacgg ccaggccagg 5880

cccaggcagg catgggtgga tcccacaggg ctctgagcta gaccggctcc acggtggccc 5940

cactacgagg ccagttctgc agtcttggcc ttgtcaccca tcgagaggct gctctgatgg 6000

ttcccagcca cacaccagct ctcctgggga aactatttct ttcgttcttt tggcctcgga 6060

gaggtccgag gcaagtacat ttcttaaaag gtaataaaat gcattattgg aaagttggac 6120

agtcaggcca cgactcctag cccacggcgt gccccacctc ccagcagccc cttcagcccc 6180

ttgcccctgt tgccccaaac ctcagggttc cctcttgcat attcatgggg gaaccacagt 6240

gctgatgtgt acttccccac tgtcagctcg gctgcacctc gtgtggcgcc aggtcccaag 6300

ggcctctgca gaggccaggc tgtgagcccc ttgcctgcct gctcccctga tgaggcaaca 6360

gcttctctga aatgagctgc cggccaggag caggcaggca ccagcctgtc tttccttttc 6420

tggtaattcc tcagcactga ggctccgttc ctgggcaccc caggattgaa gggaacctca 6480

gaatcatgtc actgccattc tagagtttca atccaagggg tcccctttag ctcatctcca 6540

agatgggtaa acgtagccac cattcagaaa gcccagaaat tcttgttccc acatcttaga 6600

cccctgagca acacaaggag aaaatgcagc tgcttaccta ttaatatcta ctgaggacaa 6660

atcagcaaag cctcaaagga gtcgtctcag gtagggtact tggcctgtgg caggagagac 6720

agaggcacaa acccacccac ccatcagctt ccggtggctg atcggggccc agggagggaa 6780

gcaggtgaaa cagcagggtg ggggtgactt ggcagtcgtg catctcctcc ccgactctga 6840

ggcctggcag gaggaggcca caccagcctc acctgccctg acccccgccc cccaccctgt 6900

gaccctgtgg ctatggccgc tggtcgccct tgtccccaaa atcaccatgc ctgtggccac 6960

gtccctcatc ttctccaaaa gcatcattaa gaacaagtga ttttggatga tggatttttg 7020

atttacaaac ggcgcatttc ccttggagtg gaacagaaag gaaaccattt aatggcgccc 7080

ttcttttcaa gcatgaatac attttaatga aactatttta ttgtatttga ggaaatggag 7140

agttgaacat tccaaccaat caatagccaa ttaattgcta taaagctaaa aagaaaataa 7200

ataaatcctg agtctatttt aaacactgca aaaagttcag agcctcagaa tctggccttc 7260

ccctccataa ggtgcacgag catgtaaaca cacacacaca cacacacaca cacacacaca 7320

cactcactca ctcccctaca cctcagacat acttgaaact cagaaacagc actgagtctc 7380

cccatgccaa ttcttgcctg ctgtcttcga cttgggtcag agaaggtgag cagacccggc 7440

agcagcctgt cccggggctc aggaagaggc aggcccatcc cctggcccca agcacccagc 7500

acaacagagg gtggcgggca gtgaggcctt ggcgttgcct gggccccact tctcagcccc 7560

agctgctggg cctccaaggt tgggctgagg atggagtttt ggctctgggt ttgccctgac 7620

tcctgctgga agacgctgcc ctggtttttc accctctagt ggccttggac attgagtatt 7680

tgtagaaatg cagattacat tgcaaatgga aacctttgcc aggaagacac atgcattttg 7740

cttttaattc tttgagacat ttgattttgt cttagggact gacctttcag catcaaagaa 7800

atacatatct actgtatccg ccaaagtttg tgatgcctgc atagacgctt acttgtaaaa 7860

aaaaaaaaat acaaaaaaat acaaaaaaac caacaacaaa aaccacaatt gaattgcctt 7920

tgaaagtggg agatgatctg tctccaacgg attgaaaaaa aaaaatgctt cttaaaaaat 7980

gtgtatgttt tgtattcttt ttttctagta gaaaataact gacttgaaat attggtggtt 8040

tttttcttag tgacgtgtgt tgcttttgtg tgtaataata tttgaatgta attacagcag 8100

tgccaatttg ccaaagatgt tggacatatt tttctttttt ggggaggagg gcagggctag 8160

gggtgggact tgggagaaaa caggggtggg gttttggttt aatttttttt ttactttttt 8220

ttccttgtca aacctgaaat ttgtggcttc cttttaagtt aaatggttga ctgcaacacc 8280

tttattttag attagttgga gaaacatgca ataagattgg cgtagtttca atatctgtgt 8340

gtcttttcat gagtggctgt tacttgtgaa gaattgattt tatgtaacct ttatgtgaga 8400

taattatttg taaatatttg ccataatttt attggttcct aaaataaaag taatttttta 8460

agttcagaaa aa 8472

<210> 7
<211> 8385
<212> RNA
<213> Homo sapiens

<400> 7
agttccttaa tcatttcacg gtgccttcgg acgctttttt tccacctaaa acgtttagtt 60

tcagctcagt gatcagctac cccagctcgg cgggggagcg gaaggcttga attattccga 120

cctgtgagcg gcccctggca ccaaaaaaaa aaaaaaaaaa aaaaaaaaaa gaaaaaaaaa 180

ggcacaaaaa agtggaaact tttccctgtc cattccatca agtcctgaaa aatcaaaatg 240

gatttagaga aaaattatcc gactcctcgg accagcagga caggacatgg aggagtgaat 300

cagcttgggg gggtttttgt gaatggacgg ccactcccgg atgtagtccg ccagaggata 360

gtggaacttg ctcatcaagg tgtcaggccc tgcgacatct ccaggcagct tcgggtcagc 420

catggttgtg tcagcaaaat tcttggcagg tattatgaga caggaagcat caagcctggg 480

gtaattggag gatccaaacc aaaggtcgcc acacccaaag tggtggaaaa aatcgctgaa 540

tataaacgcc aaaatcccac catgtttgcc tgggagatca gggaccggct gctggcagag 600

cgggtgtgtg acaatgacac cgtgcctagc gtcagttcca tcaacaggat catccggaca 660

aaagtacagc agccacccaa ccaaccagtc ccagcttcca gtcacagcat agtgtccact 720

ggctccgtga cgcaggtgtc ctcggtgagc acggattcgg ccggctcgtc gtactccatc 780

agcggcatcc tgggcatcac gtcccccagc gccgacacca acaagcgcaa gagagacgaa 840

ggtattcagg agtctccggt gccgaacggc cactcgcttc cgggcagaga cttcctccgg 900

aagcagatgc ggggagactt gttcacacag cagcagctgg aggtgctgga ccgcgtgttt 960

gagaggcagc actactcaga catcttcacc accacagagc ccatcaagcc cgagcaggct 1020

ccccctacta ttatagcgct gccgcccgag gagccgcccc acctgcagcc gccactgcct 1080

atgaccgtca ctgacccttg gagccaggcg ggcaccaaac actgatggca cctattgagg 1140

gtgacagcca cccagccctc ctgaagatag ccagagagcc catgagaccg tcccccagca 1200

tcccccactt gcctgaagct cccctcttcc tctcttcctc cagggactct ggggcccttt 1260

ggtggggccg ttggacttct ggatgcttgt ctatttctaa aagccaatct atgagcttct 1320

cccgatggcc actgggtctc tgcaaaccaa tagactgtcc tgcaaataac cgcagcccca 1380

gcccagcctg cctgtcctcc agctgtctga ctatccatcc atcataacca ccccagcctg 1440

ggaaggagag cttgcttttg ttgcttcagc agcacccatg taaatacctt cttgcttttc 1500

tgtgggcctg aaggtccgac tgagaagact gctccaccca tgatgcatct cgcactcttg 1560

gtgcatcacc ggacatctta gacctatggc agagcatcct ctctgccctg ggtgaccctg 1620

gcaggtgcgc tcagagctgt cctcaagatg gaggatgctg cccttgggcc ccagcctcct 1680

gctcatccct ccttctttag tatctttacg aggagtctca ctgggctggt tgtgctgcag 1740

gctccccctg aggcccctct ccaagaggag cacactttgg ggagatgtcc tggtttcctg 1800

cctccatttc tctgggaccg atgcagtatc agcagctctt ttccagatca aagaactcaa 1860

agaaaactgt ctgggagatt cctcagctac ttttccgaag cagaatgtca tccgaggtat 1920

tgattacatt gtggactttg aatgtgaggg ctggatggga cgcaggagat catctgatcc 1980

cagccaagga ggggcctgag gctctcccta ctccctcagc ccctggaacg gtgttttctg 2040

aggcatgccc aggttcaggt cacttcggac acctgccatg gacacttcac ccaccctcca 2100

ggaccccagc aagtggattc tgggcaagcc tgttccggtg atgtagacaa taattaacac 2160

agaggacttt cccccacacc cagatcacaa acagcctaca gccagaactt ctgagcatcc 2220

tctcggggca gaccctcccc gtcctcgtgg agcttagcag gcagctgggc atggaggtgc 2280

tggggctggg gcagatgcct aatttcgcac aatgcatgcc cacctgttga tctaaggggc 2340

cgcgatggtc agggccacgg ccaaggccca cgggaacttg gagagggagc ttggagaact 2400

cactgtgggc tagggtggtc agaggaagcc agcagggaag atctggggga cagaggaagg 2460

cctcctgagg gaggggcagg agagcagtga ggagctgctg tgtgacctgg gagtgatttt 2520

gacatggggg tgccaggtgc catcatctct ttacctgggg ccttaattcc ttgcatagtc 2580

tctcttgtca agtcagaaca gccaggtaga gcccttgtcc aaacctgggc tgaatgacag 2640

tgatgagagg gggcttggcc ttcttaggtg acaatgtccc ccatatctgt atgtcaccag 2700

gatggcagag agccagggca gagagagact ggacttggga tcagcaggcc aggcaggtct 2760

tgtcctggtc ctggccacat gtctttgctg tgggacctca gacaaaaccc tgcacctctt 2820

tgagccttgg ctgccttggt gcagcagggt catctgtagg gccaccccac agctctttcc 2880

ttcccctcct ctctccaggg agccggggct gtgagaggat catctggggc aggccctcca 2940

cttccaagca agcagatggg ggtggcacct tgaggcccaa taatatttgg accaagtggg 3000

aaacaagaac actcggaggg gcgggaatca gaagagcctg gaaaaagacc tagcccaact 3060

tcccttgtgg gaaactgagg cccagcttgg ggaaggccag gaccatgcag ggagaaaaag 3120

cagacttcct ctggccaccg ctaagtattt tgttccctaa gtcccccaca gggtggtgga 3180

acagaagaga aaactaaggc tcagcaaggt ggagtcctca agcagtgact ggtgggggtg 3240

gggctgggac tcgggctcct gacccccaac ccatggtgtt ccctgtcacc ctggtctatc 3300

cacatctcct attcctgagg agagttgaca gtaagagcag tgagagatgg ttctgggccc 3360

catgccctag acaatcagtc tgtaagaact gccaaggaag cctggtcacc caggccaggg 3420

atggagccca gcgagctcac agcaggcaca tgcacccccg cccccaccca gaacctgcgg 3480

ggcaaagaag ggaggtagtt ggggccagag tccacctcca ggagccaggg tgagctggct 3540

gcagcttcca cctgtcaggt aaggtaggag aaggtatgtt acctggcatc tctctccctg 3600

cctccctcca tttagcagga agtggtgggg tcaggggtct tcagcacaga cttcttgagc 3660

ctctgccccc tgtcaccctt cttttggaat gatgtgtacc cacatctttg gatcctgccc 3720

tccttgtggt tccaggctgt tgggaggagg tcagcctccc ctgaaccagc tgcctgaggc 3780

atgcagcatc cttcctcggc aaagcccacc tggctcacag cggccccctc tccccatcct 3840

gttcctcttc ttgccctgta atgagctccc cccatacctt tcctccctca cctgaggctt 3900

tgctggtcct cagattggtt ttgtatttgt gagacaacca cttgactcct gggctgccag 3960

gcggaagcac aagcgcacat ggatgcacac ggatgttctc acacacacac gcccgctctc 4020

accaattcac agcacctcgt ggtccagcgg agctgcctgg gagcttggtg aggatggctc 4080

caaaggacac aagccgttga gtagatgcca gagaattctg aatggaaaac acaagtccgg 4140

ggcctcacca gcatcgtggc agaaggcctg gggcatttct ccatgggcct tcttccctgt 4200

gttcgagctc tgacttttgg aaaaggacat tgtggatttt atgaaaattt ctcataccat 4260

cagtctagct ccaacctaga aaaattggat gatatatcaa acccaacatc cctttcccaa 4320

ggcaccttag ttaaggctag cccttccata actgacattg tacggtgctt tgcaatcctc 4380

aaccactctg tggagcaaag agcatgatgc ctactttata gacggggaaa ttgacatttg 4440

gggctctcac ggcaacatag aggcgaagtg aattctcaga tctccaggcc ccattctctc 4500

tccaccgagt tgtgctcctg tcatgggagt gtatggctta aagacactcc cccaccccca 4560

ttccctagaa atcccccaga cccacaatca ggcaagaaag aacaggaccc cagaggctgg 4620

cctgacctag gggcctgcag gttggcggct ttgtttccta agaacattga aacctgcgga 4680

gtctttgacc aaatccacaa cagtgcttct gaggtttcat ccagactctt tcccagctgt 4740

ccctgaggtt cagagggtat cagagtcaat tcaaggccat gccataatcc ctgaccaggc 4800

ctggacatgg gtccagccct gactccaggg gtccaggtgc caaggtcatg tgctgtcccc 4860

cacttccctt tctttgcctt cgcactttgg aacaggctcc aggcctggct gtgacagtat 4920

gcaggagtgc ccaagccagg gccaccaggg tgtccacagc ccctggaaac acagatcaca 4980

atctcaagtc ccctgaatga actgcttcct gggtgaacgg ggtggtgtag ccttgccact 5040

cgggcagcgc accagacagt acggtgcagc agtgcccgag atgcccagaa gtgtgcctgc 5100

ccccctgagt ggcattcaag tatgaaaact tgtaaaattt tctgtcggcc taaagaaaaa 5160

tgtccatggg ccaaacttga cccactgggc accagcctgt gagccccaat gcctgtcaat 5220

tgcccccttt ctcattcacc tcctgtcctt tgttgcagat ttggaggaga tgggagccca 5280

gagaggtgag agatgtgctt cagggtgtcc agcaaggcag gggtagaaac gggcacgccc 5340

agagcctgcc actctcccag gcctcatctt gggctcttgc aagtcttgcg ctttgaagat 5400

ggagttgggt ggaaggtcag aggcgctggg gacgggattg gggagctgct gggttttcag 5460

gggaggtcag tgctgcttgg gcacctttca catgtgcagg gaagagactc agatgtggcc 5520

acagggcact gaggggtgaa atccatttac cagaagtcac gctccagaac gactgcccag 5580

ccttggcagc cagtggctcc agccaccccc tcctcatgac attcagccag aattgagctc 5640

caagccaggg aagcaaatct taaaaaccaa ccaagcaccc tgacacagcc ctagaaacac 5700

gatgagtctg aaatacagct tcccaggagg ggagtctaag atacagcttc ccgggagggt 5760

ggaaaccaac tcctgcccca cggccaggcc aggcccaggc aggcatgggt ggatcccaca 5820

gggctctgag ctagaccggc tccacggtgg ccccactacg aggccagttc tgcagtcttg 5880

gccttgtcac ccatcgagag gctgctctga tggttcccag ccacacacca gctctcctgg 5940

ggaaactatt tctttcgttc ttttggcctc ggagaggtcc gaggcaagta catttcttaa 6000

aaggtaataa aatgcattat tggaaagttg gacagtcagg ccacgactcc tagcccacgg 6060

cgtgccccac ctcccagcag ccccttcagc cccttgcccc tgttgcccca aacctcaggg 6120

ttccctcttg catattcatg ggggaaccac agtgctgatg tgtacttccc cactgtcagc 6180

tcggctgcac ctcgtgtggc gccaggtccc aagggcctct gcagaggcca ggctgtgagc 6240

cccttgcctg cctgctcccc tgatgaggca acagcttctc tgaaatgagc tgccggccag 6300

gagcaggcag gcaccagcct gtctttcctt ttctggtaat tcctcagcac tgaggctccg 6360

ttcctgggca ccccaggatt gaagggaacc tcagaatcat gtcactgcca ttctagagtt 6420

tcaatccaag ggtccccctt tagctcatct ccaagatggg taaacgtagc caccattcag 6480

aaagcccaga aattcttgtt cccacatctt agacccctga gcaacacaag gagaaaatgc 6540

agctgcttac ctattaatat ctactgaggg acaatcagca aagcctcaaa ggagtcgtct 6600

caggtagggt acttggcctg tggcaggaga gacagaggca caaacccacc cacccatcag 6660

cttccggtgg ctgatcgggg cccagggagg gaagcaggtg aaacagcagg gtgggggtga 6720

cttggcagtc gtgcatctcc tccccgactc tgaggcctgg caggaggagg ccacaccagc 6780

ctcacctgcc ctgacccccg ccccccaccc tgtgaccctg tggctatggc cgctggtcgc 6840

ccttgtcccc aaaatcacca tgcctgtggc cacgtccctc atcttctcca aaagcatcat 6900

taagaacaag tgattttgga tgatggattt ttgatttaca aacggcgcat ttcccttgga 6960

gtggaacaga aaggaaacca tttaatggcg cccttctttt caagcatgaa tacattttaa 7020

tgaaactatt ttattgtatt tgaggaaatg gagagttgaa cattccaacc aatcaatagc 7080

caattaattg ctataaagct aaaaagaaaa taaataaatc ctgagtctat tttaaacact 7140

gcaaaaagtt cagagcctca gaatctggcc ttcccctcca taaggtgcac gagcatgtaa 7200

acacacacac acacacacac acacacacac acacactcac tcactcccct acacctcaga 7260

catacttgaa actcagaaac agcactgagt ctccccatgc caattcttgc ctgctgtctt 7320

cgacttgggt cagagaaggt gagcagaccc ggcagcagcc tgtcccgggg ctcaggaaga 7380

ggcaggccca tcccctggcc ccaagcaccc agcacaacag agggtggcgg gcagtgaggg 7440

cctggcgttg cctgggcccc acttctcagc cccagctgct gggcctccaa ggttgggctg 7500

aggatggagt tttggctctg ggtttgccct gactcctgct ggaagacgct gccctggttt 7560

ttcaccctct agtggccttg gacattgagt atttgtagaa atgcagatta cattgcaaat 7620

ggaaaccttt gccaggaaga cacatgcatt ttgcttttaa ttctttgaga catttgattt 7680

tgtcttaggg actgaccttt cagcatcaaa gaaatacata tctactgtat ccgccaaagt 7740

ttgtgatgcc tgcatagacg cttacttgta aaaaaaaaaa aatacaaaaa aatacaaaaa 7800

aaccaacaac aaaaaccaca attgaattgc ctttgaaagt gggagatgat ctgtctccaa 7860

cggattgaaa aaaaaaaatg cttcttaaaa aatgtgtatg ttttgtattc tttttttcta 7920

gtagaaaata actgacttga aatattggtg gtttttttct tagtgacgtg tgttgctttt 7980

gtgtgtaata atatttgaat gtaattacag cagtgccaat ttgccaaaga tgttggacat 8040

atttttcttt tttggggagg agggcagggc taggggtggg acttgggaga aaacaggggt 8100

ggggttttgg tttaattttt tttttacttt tttttccttg tcaaacctga aatttgtggc 8160

ttccttttaa gttaaatggt tgactgcaac acctttattt tagattagtt ggagaaacat 8220

gcaataagat tggcgtagtt tcaatatctg tgtgtctttt catgagtggc tgttacttgt 8280

gaagaattga ttttatgtaa cctttatgtg agataattat ttgtaaatat ttgccataat 8340

tttattggtt cctaaaataa aagtaatttt ttaagttcag aaaaa 8385

<210> 8
<211> 34
<212> DNA
<213> Homo sapiens

<400> 8
cgagctctcc arcacagcct wcatgcarct carc 34

<210> 9
<211> 34
<212> DNA

<213> Homo sapiens

<400> 9
gtctagattc tcacaagagt ccgatagacc ctgg                                    34

<210> 10
<211> 34
<212> DNA
<213> Homo sapiens

<400> 10
cggtaccaag aasamcctgt wcctgcaaat gasc                                    34

<210> 11
<211> 34
<212> DNA
<213> Homo sapiens

<400> 11
gtctagattc tcacaagagt ccgatagacc ctgg                                    34

<210> 12
<211> 34
<212> DNA
<213> Homo sapiens

<400> 12
cggtaccaga ctgarcatca scaaggacaa ytcc                                    34

<210> 13
<211> 34
<212> DNA
<213> Homo sapiens

<400> 13
gtctagattc tcacaagagt ccgatagacc ctgg                                    34

<210> 14
<211> 21
<212> DNA
<213> Homo sapiens

<400> 14
ttcaaagcac aatgcctggc t                                                  21

<210> 15
<211> 34
<212> DNA
<213> Homo sapiens

<400> 15
gtctagattc tcacaagagt ccgatagacc ctgg                                    34

<210> 16
<211> 28
<212> DNA
<213> Homo sapiens

<400> 16
tggccatggg ctgcctagcc cgggactt                                           28

<210> 17
<211> 26
<212> DNA
<213> Homo sapiens

<400> 17
gcctgactga gctcacacaa ggagga                                             26

## Description

### FIELD OF THE INVENTION

The present invention relates to methods and compositions for use in the treatment and/or prevention of hematologic malignancies. More specifically, it concerns the use of an interleukin-6 (IL-6) inhibitor for the prevention and treatment of hematologic malignancies in a patient.

### BACKGROUND OF THE INVENTION

IL-6 is a pleiotropic pro-inflammatory cytokine produced by a variety of cell types, including stimulated fibroblasts, monocytes and endothelial cells. Other cells, such as T cells, B-cells, macrophages, keratinocytes and osteoblasts, can produce IL-6 on stimulation.

IL-6 is an important factor in a variety of human diseases, including cardiovascular conditions, sepsis, fever, cachexia, insulin resistance, osteoporosis, and neurologic disorders. Most notably, increased production of IL-6 contributes to the pathogenesis of many chronic inflammatory and autoimmune diseases.

A variety of anti-human IL-6 antagonistic antibodies have thus been developed as potential therapies for these IL-6 related diseases. The positive effect of inhibiting IL-6 signalling in cancer and inflammatory diseases has been demonstrated for example by the use of a humanized anti-IL-6Ra antibody tocilizumab, an antibody directed towards the IL-6 receptor. Treatment of patients with this antibody has proven effective in a number of diseases including rheumatoid arthritis, juvenile idiopathic arthritis, Crohn's disease and systemic lupus erythematosus.

Several other biologicals have also been approved for the treatment of different conditions, including siltuximab (Sylvant), an antibody directed against IL-6 itself approved for treatment of HHV-8-negative patients with multicentric Castleman's disease, and sarilumab (Kevzara), a human monoclonal antibody against the IL-6 receptor developed for the treatment of rheumatoid arthritis.

Other than biologicals, common therapies for malignancies related to B-cell and IL-6 include: chemotherapy; targeted therapy i.e. the use of drugs targeting specific vulnerabilities in cancer cells e.g. imatinib (Gleevec) in leukemia; radiotherapy; and stem cell transplantation, but none of them take into account the genetic background of the subject and its effect on the administered treatment. Thus, there is a need in the state of the art of providing new method of treatment and diagnosis in the treatment of malignancies related to B-cell and IL-6.

### DETAILED DESCRIPTION OF THE INVENTION

The inventors have discovered that *Pax5* loss results in the enhanced production of IL-6, which appears to act in an autocrine fashion to promote leukemia growth. Specifically, *in vivo* genetic downregulation of IL-6 in *Pax5* mutant mice retards B-cell leukemogenesis, and *in vivo* pharmacologic inhibition of IL-6 with a neutralizing IL-6 antibody in *Pax5* mutant mice with B-ALL clears leukemic cells. This surprising IL-6 signalling effect has been substantiated in human subjects and confirms that aberrant IL-6 expression caused by *Pax5* loss is a hallmark of *Pax5*-dependent B-ALL, highlighting the role of IL-6 as a suitable therapeutic target for B-ALL and other diseases characterized by *PAX5* loss.

The present invention thus provides IL-6 antagonists for use in the treatment of hematologic malignancies, especially in a patient having an abnormal profile of inflammatory markers, more particularly an increased expression level of the gene encoding IL-6 with respect to a reference level.

In one aspect, the present invention relates to an IL-6 inhibitor for use in the treatment and/or prevention of hematologic malignancies comprising deficiencies in the B-cell transcription factor gene PAX5 in a subject (hereinafter, "use of the invention").

Human IL-6 is an interleukin encoded by the IL-6 gene (NCBI, Gene ID: 3569, last modified 11-Oct-2020; SEQ ID NO 1). The protein encoded by the gene is also known as BSF-2, CTL differentiation factor (CDF), hybridoma growth factor, or interferon beta-2 (IFN-beta-2) (UniProt/Swiss-Prot P05231 last modified October 7, 2020; SEQ ID NO 2).

Human IL-6 is a glycoprotein composed of 184 amino acids with a molecular weight of 21-28 kDa, depending on its degree of glycosylation. It has a 4-helix bundle structure made up of 4 long α-helices arranged in an up-up-down-down topology. The gene for IL-6 is located at chromosome 7p21, and consists of 5 exons and 4 introns

The present invention comprises the use of an IL-6 inhibitor (hereinafter "inhibitor of the invention"). In the present invention, the term "IL-6 inhibitor" refers to any molecule capable of completely or partially inhibiting the biological activity of IL-6 by any mode of action, including but not limited to preventing the expression product of the IL-6 gene from being produced (interrupting the IL-6 gene transcription and/or blocking the translation of the mRNA coming from the IL-6 gene expression) and directly inhibiting the IL-6 biological activity, for example, and among others, by binding to the interleukin or its receptor. Methods for decreasing/abrogating the expression of the gene encoding the IL-6 protein include, without being limited to, editing technologies such as CRISPR/cas9 or Cas9 nickase technology. In the context of the present invention, the terms "IL-6 antagonist" and "IL-6 inhibitor" are equivalent and can be used interchangeably throughout the present description.

IL-6 inhibitors may be identified using methods well known to the skilled person, including but not limited to competition ELISA, pSTAT3 HTRF assays or B9 cell proliferation assays.

Any IL-6 inhibitor may be used in the use of the present invention. However, in a particular embodiment of the use of the invention, the IL-6 inhibitor may be selected from the group consisting of: (i) an IL-6 antibody or fragment thereof; (ii) an IL-6 receptor antibody or fragment thereof; (iii) a small molecule; and (iv) an inhibitor of IL-6 gene expression such as siRNA, an antisense oligonucleotide, a nuclease or a ribozyme.

### IL-6 antibodies

In the context of the present invention, the antibody is an IL-6 inhibitor.

The term "IL-6 inhibitor antibody", or any variations thereof, is understood as any antibody capable of binding specifically to IL-6 and inhibiting one or more of the functions of said interleukin.

The term "antibody" is understood as a polypeptide including at least a light chain or heavy chain immunoglobulin variable region which specifically recognizes and binds an epitope of an antigen, such as IL-6, or a fragment thereof. Antibodies are composed of a heavy and a light chain, each of which has a variable region, termed the variable heavy (VH) region and the variable light (VL) region. Together, the VH region and the VL region are responsible for binding the antigen recognized by the antibody. Antibodies of the present disclosure include those that are specific for IL-6 and reduce or inhibit the biological activity of an IL-6 protein.

The term "antibody" includes intact immunoglobulins, as well the variants and portions thereof, such as Fab' fragments, F(ab)'2 fragments, single chain Fv proteins ("scFv"), and disulfide stabilized Fv proteins ("dsFv"). A scFv protein is a fusion protein in which a light chain variable region of an immunoglobulin and a heavy chain variable region of an immunoglobulin are bound by a linker, while in dsFvs, the chains have been mutated to introduce a disulfide bond to stabilize the association of the chains. The term also includes genetically engineered forms such as chimeric antibodies (for example, humanized murine antibodies), heteroconjugate antibodies (such as, diabodies or bispecific antibodies).

The antibodies can be prepared using any of the methods which are known by the person skilled in the art, some of which have been mentioned above. Thus, the polyclonal antibodies are prepared by means of immunizing an animal with the protein to be inhibited. The monoclonal antibodies are prepared using the method described by Kohler and Milstein et al. 1975 (Nature 256: 495-497). Once antibodies with IL-6 protein binding capacity are identified, those capable of inhibiting the activity of this protein will be selected using an inhibitory agent identification assay.

Examples of antibodies anti-IL6 which may be used in the context of the present invention include, without limiting to, siltuximab (Sylvant), an antibody directed against IL-6 itself.

### IL-6 receptor antibodies

In the context of the present invention, the antibody is an IL-6 receptor inhibitor. In this case, the term "IL-6 receptor inhibitor antibody" or any variations thereof is understood as any antibody capable of binding specifically to the IL-6 receptor and inhibiting one or more of the functions of said receptor. The term "antibody" has been described above, and the explanation is applicable to the present inventive aspect. Examples of IL-6 receptor inhibitor antibody which may be used in the context of the present invention include, without limiting to human monoclonal antibodies like tocilizumab, sarilumab and BCD-089.

### Small molecules

The inhibitor small molecules according to the invention are molecules, typically with a molecular weight less than about 1000 Daltons, or in some embodiments, less than about 500 Daltons, wherein the molecule is capable of modulating, to some measurable extent, an activity of a target molecule, such as IL-6.

### Antisense Oligonucleotides

An additional aspect of the invention relates to the use of isolated "antisense" nucleic acids to inhibit expression, for example, for inhibiting transcription and/or translation of a nucleic acid which encodes IL-6, the activity of which is to be inhibited. The antisense nucleic acids can be bound to the target potential of the drug by means of conventional base complementarity or, for example, in the case of binding to double stranded DNA through specific interaction in the large groove of the double helix. Generally, these methods refer to a range of techniques generally used in the art and they include any method which is based on the specific binding to oligonucleotide sequences.

An antisense oligonucleotide can be distributed, for example, as an expression plasmid which, when it is transcribed in cell, produces RNA complementary to at least one unique part of the cellular mRNA encoding IL-6. Alternatively, the antisense oligonucleotide is an oligonucleotide probe generated *ex vivo* which, when introduced into the cell, produces inhibition of gene expression hybridizing with the mRNA and/or gene sequences of a target nucleic acid. Such oligonucleotide probes are preferably modified oligonucleotides which are resistant to endogenous nucleases, for example, exonucleases and/or endonucleases and are therefore stable in vivo. Examples of nucleic acid molecules for use thereof as antisense oligonucleotides are DNA analogs of phosphoramidate, phosphothionate and methyl phosphonate.

With respect to the antisense oligonucleotide, the oligodeoxyribonucleotide regions derived from the starting site of the translation, for example, between -10 and +10 of the target gene are preferred. The antisense approximations involve the oligonucleotide design (either DNA or RNA) that are complementary to the mRNA encoding the target polypeptide. The antisense oligonucleotide will be bound to the transcribed mRNA and translation will be prevented.

The oligonucleotides which are complementary to the 5' end of the mRNA, for example the non-translated 5' sequence up to and including the start codon AUG must function in the most efficient manner to inhibit translation. Nevertheless, it has been shown that the sequences complementary to the non-translated 3' sequences of the mRNA are also efficient for inhibiting mRNA translation. Therefore, complementary oligonucleotides could be used at the non-translated 5' or 3' regions, non-coding regions of a gene in an antisense approximation to inhibit the translation of that mRNA. The oligonucleotides complementary to the non-translated 5' region of the mRNA must include the complement of the start codon AUG. The oligonucleotides complementary to the coding region of the mRNA are less efficient translation inhibitors but they could also be used according to the invention. If they are designed to hybridize with the 5' region, 3' region or the coding region of the mRNA, the antisense nucleic acids must have at least six nucleotides long and preferably have less than approximately 100 and more preferably less than approximately 50, 25, 17 or 10 nucleotides long.

Preferably, *in vitro* studies are performed first to quantify the capacity of the antisense oligonucleotides for inhibiting gene expression. Preferably these studies use controls which distinguish between antisense gene inhibition and non-specific biological effects of the oligonucleotides. Also, preferably these studies compare the levels of target RNA or protein with that of an internal control of RNA or protein. The results obtained using the antisense oligonucleotides can be compared with those obtained using a control oligonucleotide. Preferably the control oligonucleotide is approximately of the same length as the oligonucleotide to be assayed and the oligonucleotide sequence does not differ from the antisense sequence more than it is deemed necessary to prevent the specific hybridization to the target sequence.

The antisense oligonucleotide can be a single or double stranded DNA or RNA or chimeric mixtures or derivatives or modified versions thereof. The oligonucleotide can be modified in the base group, the sugar group or the phosphate backbone, for example, to improve the stability of the molecule, its hybridization capacity, etc. The oligonucleotide may include other bound groups, such as peptides (for example, for directing them to the receptors of the host cells), agents for facilitating transport through the cell membrane or the blood-brain barrier, and intercalating agents. For this purpose, the oligonucleotide can be conjugated to another molecule, for example, a peptide, a transporting agent, hybridization triggered cleaving agent, etc.

The antisense oligonucleotides may comprise at least one group of modified base. The antisense oligonucleotide may also comprise at least a modified sugar group selected from the group including but not limited to arabinose, 2-fluoroarabinose, xylulose, and hexose. The antisense oligonucleotide may also contain a backbone similar to a neutral peptide. Such molecules are known as peptide nucleic acid (PNA) oligomers. In yet another embodiment, the antisense oligonucleotide comprises at least one modified phosphate backbone. In yet another embodiment, the antisense oligonucleotide is an alpha-anomeric oligonucleotide.

While antisense oligonucleotides complementary to the coding region of the target mRNA sequence can be used, those complementary to the transcribed non translated region can also be used.

In some cases, it may be difficult to reach the sufficient intracellular concentrations of the antisense to suppress the endogenous mRNA translation. Therefore, a preferred approximation uses a recombinant DNA construct in which the antisense oligonucleotide is placed under the control of a strong pol III or pol II promoter.

Alternatively, the target gene expression can be reduced by directing deoxyribonucleotide sequences complementary to the gene regulating region (i.e., the promoter and/or enhancers) to form triple helix structures preventing gene transcription in the target cells in the body. In certain embodiments, the antisense oligonucleotides are antisense morpholines.

### siRNA

Small interference RNA or siRNA are agents which are capable of inhibiting the expression of a target gene by means of RNA interference. A siRNA can be chemically synthesized, can be obtained by means of *in vitro* transcription or can be synthesized *in vivo* in the target cell. Typically, the siRNA consists of a double stranded RNA between 15 and 40 nucleotide long and may contain a 3' and/or 5' protruding region of 1 to 6 nucleotides. The length of the protruding region is independent of the total length of the siRNA molecule. The siRNA acts by means of degrading or silencing the target messenger after transcription.

The siRNA of the invention is substantially homologous to the mRNA of the IL-6 encoding gene or to the gene sequence which encodes said protein. "Substantially homologous" is understood as having a sequence which is sufficiently complementary or similar to the target mRNA such that the siRNA is capable of degrading the latter through RNA interference. The siRNA suitable for causing said interference include siRNA formed by RNA, as well as siRNA containing different chemical modifications such as: (i) siRNA in which the bonds between the nucleotides are different than those appear in nature, such as phosphorothionate bonds; (ii) Conjugates of the RNA strand with a functional reagent, such as a fluorophore; (iii) Modifications of the ends of the RNA strands, particularly of the 3' end by means of the modification with different hydroxyl functional groups in 2' position; (iv) Nucleotides with modified sugars such as 0-alkylated residues on 2' position like 2'-O-methylribose or 2'-O-fluororibose; or (v) Nucleotides with modified bases such as halogenated bases (for example 5-bromouracil and 5-iodouracil), alkylated bases (for example 7-methylguanosine).

The siRNA can be used in the form of a double stranded RNA with the aforementioned characteristics. Alternatively, the use of vectors containing the sense and antisense strand sequence of the siRNA is possible under the control of suitable promoters for the expression thereof in the cell of interest.

Vectors suitable for expressing siRNA are those in which the two DNA regions encoding the two strands of siRNA are arranged in tandem in one and the same DNA strand separated by a spacer region which, upon transcription, forms a loop and wherein a single promoter directs the transcription of the DNA molecule giving rise to shRNA.

The siRNA can be generated intracellularly from the so called shRNA (short hairpin RNA) characterized in that the antiparallel strands forming the siRNA are connected by a loop or hairpin region. The shRNAs can be encoded by plasmids or viruses, particularly retroviruses, and are under the control of a promoter. Promoters suitable for expressing shRNA are those indicated in the paragraph above for expressing siRNA.

The siRNA and shRNA of the invention can be obtained using a series of techniques known by the person skilled in the art. The region of the nucleotide sequence taken as a basis for designing the siRNA is not limiting and it may contain a region of the coding sequence (between the start codon and the end codon) or it may alternatively contain sequences of the non-translated 5' or 3' region preferably between 25 and 50 nucleotides long and in any position in 3' direction position with respect to the start codon. One way of designing a siRNA involves the identification of the AA(N19)TT motifs wherein N can be any nucleotide in the IL-6 gene sequence, and the selection of those having a high G/C content. If said motif is not found, it is possible to identify the NA(N21) motif wherein N can be any nucleotide.

### DNA Enzymes

On the other hand, the invention also contemplates the use of DNA enzymes to inhibit the expression of the IL-6 gene of the invention. DNA enzymes incorporate some of the mechanistic features of both antisense and ribozyme technologies. DNA enzymes are designed such that they recognize a particular target nucleic acid sequence similar to the antisense oligonucleotide, nevertheless like the ribozyme they are catalytic and specifically cleave the target nucleic acid.

### Ribozymes

Ribozyme molecules designed for catalytically cleaving transcription products of a target mRNA to prevent the translation of the mRNA which encodes IL-6, the activity of which is to be inhibited, can also be used. Ribozymes are enzymatic RNA molecules capable of catalyzing specific RNA cleaving. The mechanism of ribozyme action involves a specific hybridization of a ribozyme molecule sequence to a complementary target RNA followed by an endonucleolytic cleavage event. The composition of the ribozyme molecules preferably includes one or more sequences complementary to the target mRNA and the well-known sequence responsible for cleaving the mRNA or a functionally equivalent sequence. Examples of ribozymes to be used in the present invention include hammer-head ribozymes and endoribonuclease RNA (hereinafter "Cech type ribozymes").

The ribozymes can be formed by modified oligonucleotides (for example to improve the stability, targeting, etc.) and they should be distributed to cells expressing the target gene *in vivo.* A preferred distribution method involves using a DNA construct which "encodes" the ribozyme under the control of a strong constitutive pol III or pol II promoter such that the transfected cells will produce sufficient amounts of the ribozyme to destroy the endogenous target messengers and to inhibit translation. Since the ribozymes are catalytic, unlike other antisense molecules, a low intracellular concentration is required for its efficiency.

The present invention relates to an IL-6 inhibitor for use in the treatment and/or prevention of hematologic malignancies.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above).

As used herein, the term "preventing" (or "prevent" or "prevention") refers to the capacity of the IL-6 inhibitor to minimize or hinder the progression of a disease, condition or disorder in a subject.

As used herein, the term "subject" refers to human and non-human animals, such as veterinary subjects. The term "non-human animal" includes all vertebrates, e.g., mammals and non-mammals, such as non-human primates, mice, rabbits, sheep, dog, cat, horse, cow, chickens, amphibians, and reptiles. In a preferred embodiment, the subject is a human, man or woman of any age or race.

In the present invention, the condition or disorder to be treated or prevented is hematologic malignancies comprising deficiencies in the B-cell transcription factor gene PAX5.

As used herein, the term "hematologic malignancies" is used to refer to any malignancies that begin in blood-forming tissue, such as the bone marrow, or in the cells of the immune system. These malignancies are also known as blood cancer.

Examples of hematologic malignancies include, without limiting to, leukemia, non-Hodgkin lymphoma, Hodgkin lymphoma or multiple myeloma. However, in a preferred embodiment, the hematologic malignancy is leukemia. Examples of leukemia include, without limiting to, acute myeloid (or myelogenous) leukemia (AML), chronic myeloid (or myelogenous) leukemia (CML), acute lymphocytic (or lymphoblastic) leukemia (ALL) or chronic lymphocytic leukemia (CLL). In a more preferred embodiment, the leukemia is acute lymphocytic (or lymphoblastic) leukemia (ALL).

In the use of the invention, the hematologic malignancies comprise deficiencies in the B-cell transcription factor gene PAX5. The term "deficiencies in the B-cell transcription factor gene PAX5" is used to designate any instances in which the function of PAX5 in leukemic cells, is compromised by genetic or epigenetic modifications of the gene.

In some embodiments of the invention, the subject presents somatic lesions of the B-cell transcription factor gene *PAX5.*

PAX5 (paired-box domain 5) is a transcription factor which belongs to the family of paired-box domain transcription factors. The gene has 5 isoforms, the references of which are available in GenBank under the accession numbers NM_016734.3 (last updated on 22.11.18), NM_001280547.2 (last updated on 01.06.19), NM_001280548.2 (last updated on 31.05.19), NM_001280549.2 (last updated on 31.05.19) and NM_001280550.2 (last updated on 31.05.19; SEQ ID NO 3 to 7, respectively). It plays a dual role in the hematopoietic system; Pax5 expression is essential in B-cell precursors for normal differentiation and maturation of B-cells, but it also inhibits the differentiation and progress toward other lineages.

The somatic mutations of the gene *PAX5* may be determined using any of the standard sequencing techniques known to the skilled person; the nucleic acid may be obtained from any type of biological sample from the subject, as described above.

In another preferred embodiment of the use of the invention, the IL-6 inhibitor is comprised within a pharmaceutical composition in a therapeutically effective amount. The term "therapeutically effective amount" means the amount of IL-6 inhibitor that will elicit the biological or medical response of a tissue, system, animal or human that is being sought by the researcher, veterinarian, medical doctor or other clinician. In the context of the present invention, the biological or medical response to elicit is the treatment and/or prevention of hematologic malignancies.

The IL-6 inhibitors of the present invention can be incorporated into pharmaceutical compositions for its administration to the subject. The IL-6 inhibitors of the present invention may conveniently be presented in dosage unit form and may be prepared by any of the methods well known in the art of pharmacy. All methods include the step of bringing the active ingredient into association with the carrier which constitutes one or more accessory ingredients. In general, the pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active compound is included in an amount sufficient to produce the desired effect upon the process or condition of diseases. As used herein, the term "composition" is intended to encompass a product comprising the specified ingredients in the specified amounts, as well as any product which results, directly or indirectly, from combination of the specified ingredients in the specified amounts.

The pharmaceutical compositions containing the active ingredient may be in a form suitable for oral use, for example, as tablets, troches, lozenges, aqueous or oily suspensions, dispersible powders or granules, emulsions, solutions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. Oral tablets may also be formulated for immediate release, such as fast melt tablets or wafers, rapid dissolve tablets or fast dissolve films.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxy-propylmethylcellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions may be in the form of a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The IL-6 inhibitor of the present invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions and the like, containing the compounds of the present invention are employed. Similarly, transdermal patches may also be used for topical administration.

In the treatment, prevention, control, amelioration, or reduction of risk of hematologic malignancies, an appropriate dosage level of the IL-6 inhibitor of the invention will generally be about 0.01 to 500 mg per kg patient body weight per day which can be administered in single or multiple doses. For oral administration, the compositions may be provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1,000.0 milligrams of the IL-6 inhibitor for the symptomatic adjustment of the dosage to the patient to be treated.

It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The IL-6 inhibitor of the present invention may be used in combination with one or more other drugs in the treatment, prevention, control, amelioration, or reduction of risk of hematologic malignancies, where the combination of the drugs together are safer or more effective than either drug alone. Thus, in particular embodiment of the use of the invention, the pharmaceutical composition further comprises a compound for the treatment and/or prevention of hematologic malignancies in a subject.

Examples of other compounds useful for treating hematologic malignancies which may be used in conjunction with IL-6 inhibitor include, without being limited to, Vincristine or liposomal vincristine (Marqibo); Daunorubicin (daunomycin) or doxorubicin (Adriamycin); Cytarabine (cytosine arabinoside, ara-C); L-asparaginase or PEG-L-asparaginase (pegaspargase or Oncaspar); 6-mercaptopurine (6-MP); Methotrexate; Cyclophosphamide; Prednisone; Dexamethasone; and Nelarabine (Arranon).

The IL-6 inhibitors of the present invention may be administered by oral, parenteral (e.g., intramuscular, intraperitoneal, intravenous, ICV, intracisternal injection or infusion, subcutaneous injection, or implant), by inhalation spray, nasal, vaginal, rectal, sublingual, buccal or topical routes of administration and may be formulated, alone or together, in suitable dosage unit formulations containing conventional non-toxic pharmaceutically acceptable carriers, adjuvants and vehicles appropriate for each route of administration. In addition to the treatment of warm-blooded animals the IL-6 inhibitors of the invention are effective for use in humans.

Such other compound(s)/drug(s) may be administered by a route and in an amount commonly used therefore, contemporaneously or sequentially with the IL-6 inhibitor. When the IL-6 inhibitor is used contemporaneously with one or more other drugs, a pharmaceutical composition in unit dosage form containing such other drugs and the IL-6 inhibitor is preferred. However, the combination therapy may also include therapies in which the IL-6 inhibitor and one or more other drugs are administered on different overlapping schedules. It is also contemplated that when used in combination with one or more other active ingredients, the IL-6 inhibitor and the other active ingredients may be used in lower doses than when each is used singly. Accordingly, the pharmaceutical compositions of the present invention include those that contain one or more other active ingredients, in addition to an IL-6 inhibitor.

As explained at the beginning of the present description, the subject presents an abnormal profile of inflammatory markers, including but not limited to, an increased expression level of the gene encoding IL-6 with respect to a reference level in control individuals. Thus, IL-6 may be used as biomarker for diagnosing hematologic malignancies in a subject, in particular in malignancies comprising deficiencies in the B-cell transcription factor gene PAX5, or for determining if a subject suffering from said malignancy is susceptible of being treated with an IL-6 inhibitor. The deficiencies in B-cell transcription factor gene PAX5 may comprise somatic lesions of the B-cell transcription factor gene *PAX5,*

Thus, in another aspect, the present invention relates to an *in vitro* method for diagnosing hematologic malignancies in a subject, or for designing a personalized therapy for a subject with hematologic malignancies, hereinafter "method of the invention", comprising
(i) determining the expression levels of the gene encoding IL-6 in a biological sample from the subject, and
(ii) comparing the expression levels obtained in step (i) with a reference level, wherein if the expression levels of said gene are increased with respect to the reference level, then the subject is suffering from a hematologic malignancy or is susceptible to receive a therapy based on an IL-6 inhibitor.

All the relevant terms have been explained above in the present description, and these explanations, as well as its particular embodiments, are applicable to the present inventive aspect.

Methods for determining the expression levels of a gene are widely known in the state of the art, and any of them can be used in the context of the present invention. The determination of the expression levels of gene encoding the IL-6 protein may comprise measuring the level of cDNA, the level of mRNA, and/or the level of the protein encoded by said gene. By way of illustration and not limitation, the levels of mRNA of said gene can be quantified by means of using conventional methods, for example, methods comprising the amplification of mRNA and the quantification of the product of the amplification of said mRNA, such as electrophoresis and staining, or alternatively, by means of Southern blot and the use of suitable probes, Northern blot and the use of specific probes of mRNA of the gene of interest (gene encoding the IL-6 protein) or of the corresponding cDNA thereof, S1 nuclease mapping, hybridization, RT-Q-PCR, the micro-arrays and RNA sequencing, etc. If the quantification of the expression of gene encoding the IL-6 protein is going to be carried out from the protein, i.e. the IL-6 protein, then the isolated biological sample of the subject must be treated to extract the proteins. Methods for extracting or isolating proteins are known by the person skilled in the art and they are commercially available. The levels of gene encoding the IL-6 protein can be quantified by means of any conventional method that enables said protein to be detected and quantified in a sample of a subject. By way of illustration and not limitation, the levels of said protein can be quantified, for example, by means of the use of antibodies with the capacity to bind specifically to the IL-6 protein and the subsequent quantification of the complexes formed.

The term "reference level" refers to the expression levels of the gene encoding the IL-6 in a sample of a subject not suffering from hematologic malignancies ("control individual/s").

The levels of expression may be determined using any type of biological sample from the subject. As used herein, the term "biological sample" refers to any material comprising a nucleic acid. Examples of biological samples include, but without limiting to, a biopsy sample, a tissue (e.g. brain tissue), cell or fluid (e.g. serum, saliva, semen, sputum, cerebral spinal fluid (CSF), tears, mucus, sweat, brain extracts and the like) and blood (e.g. PBMCs (peripheral blood-derived mononuclear cells) such as neutrophils, monocytes).

Once steps (i) and (ii) of the method of the invention have been carried out, the skilled person in the art can conclude that if the expression levels of said gene IL-6 are increased with respect to the reference level, then the subject is suffering from a hematologic malignancy or is susceptible to receive a therapy based on an IL-6 inhibitor.

In a preferred embodiment of the method of the invention, the hematologic malignancies comprise deficiencies in the B-cell transcription factor gene PAX5. The deficiencies in B-cell transcription factor gene PAX5 may comprise somatic lesions of the B-cell transcription factor gene PAX5.These particular embodiments has been previously explained in the present description and it is applicable to the present inventive aspect. In another preferred embodiment, the subject is a human subject. As cited above, if the expression levels of said gene IL-6 are increased with respect to the reference level, then the subject is susceptible to receive a therapy based on an IL-6 inhibitor. Thus, in another preferred embodiment of the method of the invention, the IL-6 inhibitor is selected from the group consisting of (i) an IL-6 antibody or fragment thereof; (ii) an IL-6 receptor antibody or fragment thereof; (iii) a small molecule; and (iv) an inhibitor of IL-6 gene expression. In a more preferred embodiment, the IL-6 inhibitor is a siRNA, an antisense oligonucleotide, a nuclease or a ribozyme. All these IL-6 inhibitors have been defined and explained for the use of the invention, and, as understood by the skilled person, all of them are applicable to the present method of the invention.

In another preferred embodiment of the method of the invention, the hematologic malignancy is leukemia, preferably, the leukemia is an acute lymphoblastic leukemia. These terms have been explained in previous paragraphs of the present description.

The *in vitro* method may further comprise determining the presence of somatic lesions of the B-cell transcription factor gene PAX5, wherein if the subject presents somatic lesions of the B-cell transcription factor gene PAX5 then the subject is susceptible to receive a therapy based on an IL-6 antagonist.

The present *in vitro* method(s) can be applied to any type of biological sample or nucleic acid obtained from a biological sample from a subject. The term "biological sample" has been described above, and the explanation is applicable to the present inventive aspect

In further inventive aspects, the present invention also encompasses a method of treating or preventing hematologic malignancies in a subject, comprising reducing or inhibiting IL-6 activity in the subject, as described herein, and the use of an IL-6 inhibitor as described herein in the manufacture of a medicament for the treatment and/or prevention of hematologic malignancies in a subject. All the preferred embodiments and explained of the terms used in these inventive aspects have been explained above.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

Figure 1. IL-6 serum levels in mice and humans with B-ALL.
Figure 2. IL-6 expression in B-cells.
Figure 3. Development of natural infection-driven B-ALL in mice.
Figure 4. Mouse tumour exome sequencing in *IL-6+*/*-, Pax5+*/*-* B-ALL.
Figure 5. Inhibitory effect of anti-IL-6 antibody in *Pax5+*/*-* leukemic mice.

### EXAMPLES

### I. MATERIALS AND METHODS

### Mouse model for natural infection-driven leukemia

*Pax5+*/*-* mice (Urbanek, Wang et al. 1994 Cell 79(5):901-12) were crossed with *IL-6-*/- mice (Kopf, Baumann et al. 1994 Nature 368(6469):339-42) to generate *Pax5+*/*-*/*IL-6+*/*-* mice. These *Pax5+*/*-*/ *IL-6+*/*-* and *Pax5+*/*-* mice were bred and maintained in the SPF area of the animal house until the moment when they were relocated to an environment where natural infectious agents were present, as previously described (Martin-Lorenzo, Hauer et al. 2015 Cancer Discov 5(12):1328-43). All mouse experiments were performed following the applicable Spanish and European legal regulations, and had been previously authorized by the pertinent institutional committees of both University of Salamanca and Spanish Research Council (CSIC). *IL-6+*/*-, IL-6-*/*-, Pax5+*/*-*/*IL-6+*/*-,* and *Pax5+*/*-* mice of a mixed C57BL/6× CBA background were used in this study, with approximately equal representation of both males and females. For the experiments, *IL-6+*/*-, IL-6-*/*-, Pax5+*/*-*/*IL-6 +*/*-,* and *Pax5+*/*-* mice of the same litter were used. When the animals showed evidences of illness, they were humanely killed, and the main organs were extracted by standard dissection. All major organs were macroscopically inspected under the stereo microscope, and then representative samples of tissue were cut from the freshly dissected organs, and were immediately fixed. Differences in Kaplan-Meier survival plots of transgenic and WT mice were analysed using the log-rank (Mantel-Cox) test.

### Flow cytometry and cell sorting

Total mouse BM cells were obtained by washing the long bones with PBS with 1% FCS, using a 27-G needle. Cells were also collected from peripheral blood, and from the thymus and spleen after disrupting these organs by passing them through a 70-µm cell strainer. Erythrocytes were osmotically lysed using RCLB buffer, and nucleated cells were then washed with PBS-1% FCS. Cells were stained with the appropriate antibodies against the indicated cellular markers for 20 min at 4 °C, washed once with PBS-1% FCS, and finally they were resuspended in PBS-1% FCS with 10 µg/mL propidium iodide (PI) to exclude dead cells during data acquisition; this was performed in an AccuriC6 Flow Cytometer, and data files were analysed using Flowjo software. For this analysis, gates were set by employing the commonly used forward and perpendicular light-scattering properties of mouse hematopoietic cells, and the specific fluorescence of the staining dyes used [FITC, PE, PI, and APC excited at 488 nm (0.4 W) and 633 nm (30 mW), respectively].

The nonspecific binding of staining antibodies to the Fc receptors of immune cells was prevented by incubating the samples with anti-CD16/CD32 Fc-block solution (clone 2.4G2, cat. #553142, BD Biosciences) for 20 min at 4 °C, previously to the addition of the staining antibodies. For each sample tube, a minimum of 50,000 living (i.e. PI-negative) cells were acquired and analysed. The antibodies used for flow cytometry were all from BD Biosciences: anti-B220 345 (clone RA3-6B2, cat. #103212, used in 1:100 dilution), CD4 (clone RM4-5, cat. #100516, used in 1:250 dilution), CD8a (clone 53-6.7, cat. #100708, used in 1:250 dilution), CD11b/Mac1 (clone M1/70, cat. #553310, used in 1:200 dilution), CD19 (clone 1D3, cat. #152404, used in 1:100 dilution), CD117/c-Kit (clone 2B8, cat. 105807, used in 1:200 dilution), Ly-6G/Gr1 (clone RB6-8C5; cat. #108412, used in 1:100 dilution), IgM (clone R6-60.2, cat. #406509, used in 1:100 dilution) and CD25 (clone PC61, cat. #553866, used in 1:100 dilution).

### Real- time PCR quantification (Q-PCR) of mIL-6, mBcl6 and mBlnk

The expression of *mIL-6, mBcl6 and mBlnk* in leukemic *Pax5+*/*-* cells, healthy *Pax5+*/*-* and WT proB-cells was analysed by Q-PCR as follows: cDNA was synthesized using reverse transcriptase (Access RT-PCR System; 356 Promega, Madison, WI) and genomic DNA was removed by DNAase treatment (Roche, 04 716 728 001). Real-time PCR reactions were performed in an Eppendorf MasterCycler Realplex machine. Commercially available assays for quantitative PCR from IDT (Integrated DNA Technologies) were used: *mIL-6* (Assay ID: Mm.PT.58.10005566), *mBcl6* (Assay ID: Mm.PT.58.32669842), *mBlnk* (Assay ID: Mm.PT.58.7821272) and *Gapdh* (Assay ID: Mm.PT.39a.1). Probes were specifically designed to prevent detection of genomic DNA by PCR. Measurement of *Gapdh* gene product expression was used as an endogenous control and the total bone marrow of a WT mouse was used as a reference. All samples were run in triplicate. The comparative CT Method (ΔΔCt) was used to calculate relative expression of the transcript of interest and a positive control. The change in threshold cycle (ΔCt) of each sample was calculated as the Ct value of the tested gene (target) minus the Ct value of *Gadph* (endogenous control). The ΔΔCt of each sample was obtained by subtracting the ΔCt value of the reference from the ΔCt value of the sample. The ΔCt reference value used was the ΔCt obtained from total BM of a WT mouse. The fold change in each group, calculated as 2-ΔΔCt sample, was compared.

### Histology

Animals were sacrificed by cervical dislocation; tissue samples were formalin-fixed and included in paraffin. Pathology assessment was performed on haematoxylineosin stained sections.

### Quantification of cytokine levels in serum

Serum cytokine levels were analysed using the Cytometric Bead Array immunoassay system (CBA) (BD Biosciences) which assesses simultaneously IL-2, IL-4, IL-6, IL-10, IL-17A, TNF alpha and IFN gamma in sera from the mice (Mouse Th1 Th2 Th17 Cytokine Kit #560485; BDB); and sera from the patients and controls (Human Th1 Th2 Th17 Cytokine Kit #560484; BDB). Data acquisition was performed on a FACSCanto II flow cytometer (BDB) using the FACSDiva^{™} software program (BDB). For the evaluation of cytokine plasma levels or cytokine secretion into the culture supernatants, 50 µl of plasma was collected. Briefly, 50 µl of the serum was incubated at room temperature for 2 h at room temperature (RT) with 50 µl of anticytokine MAb-coated beads and with 50 µl of the appropriate phycoerythrin (PE) - conjugated anticytokine antibody detector. After this incubation period, samples were washed once (5 min at 200 g) in order to remove the excess of detector antibodies. Immediately afterwards, data acquisition was performed on a FACSCanto II flow cytometer (BDB) using the FACSDiva^{™} software program (BDB).

During acquisition, information was stored for 3,000 events corresponding to each bead population analysed per sample (total number of beads >9,000). For data analysis, FCAP Array Software v3.0 program (BDB) was used.

### Anti-IL-6R treatment

Anti-IL-6R antibody (tocilizumab) was obtained from Chugai Pharmaceuticals Co. Ltd. (Shizuoka, Japan). Anti-IL-6R antibody was intraperitoneally administered at 10 mg/kg twice a week. Treatments were started after the establishment of B-ALL.

### Pro-B-cell culture

Pro-B-cells were purified from BM using magnetic-activated cell sorting, selecting with anti-B220 beads (Milteny Biotec). Pro-B-cells were maintained and expanded by culturing them in Iscove's Modified Dulbecco's Medium (IMDM) supplemented with 50 µM β- mercaptoethanol, 1 mM L-Gln, 2% heat-inactivated FCS, 1 mM penicillin-streptomycin (BioWhittaker), 0.03% (w/v) primatone RL (Sigma), and 5 ng/ml mrlL-7 (R&D Systems), in the presence of Mitomycin C-treated ST2-feeder cells. Tumor pro-B-cells that could grow independently of IL-7 were grown in the same medium without this cytokine.

### Transplantation

IL-7-independent leukemic pro-B-cells were intravenously injected into 12-week-old male syngenic mice (C57BL/6× CBA) that had previously been sublethally irradiated (4 Gy). Leukemia development in the injected mice was followed by regular analysis of peripheral blood, until the moment when leukemic blasts were detected in the blood; at this point, animals were treated with anti-IL-6R antibody.

### V(D)J recombination

Immunoglobulin rearrangements were amplified by PCR using the primers below (see Table 1). Cycling conditions consisted of an initial heat-activation at 95ºC followed by 31-37 cycles of denaturation for 1 min at 95ºC, annealing for 1 min at 65ºC, and elongation for 1 min 45 s at 72ºC. This was followed by a final elongation for 10 min at 72ºC. The primer pairs are detailed in Table 1.

**Table 1**

| | | |
|---|---|---|
| SEQ ID NO 8 | V_{H}J558 forward | CGAGCTCTCCARCACAGCCTWCATGCARCTCARC |
| SEQ ID NO 9 | V_{H}J558reverse | GTCTAGATTCTCACAAGAGTCCGATAGACCCTGG |
| SEQ ID NO 10 | V_{H}7183 forward | CGGTACCAAGAASAMCCTGTWCCTGCAAATGASC |
| SEQ ID NO 11 | V_{H}7183 reverse | GTCTAGATTCTCACAAGAGTCCGATAGACCCTGG |
| SEQ ID NO 12 | V_{H}Q52 forward | CGGTACCAGACTGARCATCASCAAGGACAAYTCC |
| SEQ ID NO 13 | V_{H}Q52 reverse | GTCTAGATTCTCACAAGAGTCCGATAGACCCTGG |
| SEQ ID NO 14 | DH forward | TTCAAAGCACAATGCCTGGCT |
| SEQ ID NO 15 | DH reverse | GTCTAGATTCTCACAAGAGTCCGATAGACCCTGG |
| SEQ ID NO 16 | Cµ forward | TGGCCATGGGCTGCCTAGCCCGGGACTT |
| SEQ ID NO 17 | Cµ reverse | GCCTGACTGAGCTCACACAAGGAGGA |

### Microarray data analysis

The total RNA was first isolated using TRIzol (Life Technologies), and then it was subjected to purification with the RNeasy Mini Kit (Qiagen) using also the On-Column DNase treatment option. Quality and quantification of RNA samples were determined by electrophoresis.

Determination of the expression of the different genes in the RNA samples was performed using Affymetrix Mouse Gene 1.0 ST arrays. All bioinformatic analyses of the array data were performed using R (Team 2010 http://wwwR637projectorg/) and Bioconductor (Gentleman, Carey et al. 2004 Genome Biol 5(10): R80). First, background correction, intra- and inter-microarray normalization, and expression signal calculation using the microarray analysis algorithm (Bolstad, Irizarry et al. 2003, Bioinformatics 19(2): 185-193; Irizarry, Bolstad et al. 2003, Nucleic Acids Res 31(4): e15; Irizarry, Hobbs et al. 2003, Biostatistics 4(2): 249-264) were applied in order to determine the absolute expression signal for each gene in each array. Then, the significance analysis of microarray (SAM) (Tusher, Tibshirani et al. 2001, Proc Natl Acad Sci USA 98(9): 5116-5121) method was used to identify the gene probe sets with differential expression between experimental and control samples. SAM uses a permutation algorithm to allow to statistically infer the significance of the differential expression, and it provides *P*-values adjusted to correct for the multiple testing problem, by using FDR (Benjamini, Drai et al. 2001, Behav Brain Res 125(1-2): 279-284.). An FDR cutoff of <0.05 was used as a threshold to determine differential expression.

### Enrichment analysis

In order to identify potential signatures of gene expression associated with different biological processes, gene set enrichment analysis (GSEA) was performed using the MSigDB databases from the Broad Institute (GSEAv2.2.2) (Mootha, Lindgren et al. 2003, Genet 34(3): 267-273.) and hallmark collection of gene sets (Subramanian, Tamayo et al. 2005 Proc Natl Acad Sci USA 634 102(43): 15545-15550; Liberzon, Birger et al. 2015, Cell Syst 1(6): 417-425).

### Mouse exome library preparation and NGS

DNA was purified from samples using the AllPrep DNA/RNA Mini Kit (Qiagen) according to the manufacturer's instructions. The exome library was prepared using the Agilent SureSelectXT Mouse All Exon Kit with some modifications. Exome capture was performed by hybridization to an RNA library according to the manufacturer's protocol. Then, the captured library was purified and enriched by binding to MyOne Streptavidin T1 Dynabeads (Life Technologies) and posterior off-bead PCR amplification in the linear range. Sequencing (2 × 100 bp) was carried out in a HiSeq2500 (Illumina) using the TruSeq SBS Kit v3 with a 6-bp index read.

### Data analysis

Fastq files were generated with Illumina BcltoFastq 1.8.4. The alignment of the sequence data to the GRCm38.71 mouse reference genome was performed with BWA version 0.7.4. SAMtools was used for conversion steps and removal of duplicate reads. GATK 2.4.9 was used for local realignment around indels, SNP-calling, annotation, and recalibration. For recalibration, mouse dbSNP138 and dbSNP for the used mouse strains were used as training data sets. The variation calls obtained in this way were then annotated using the v70 Ensembl database with variant effect predictor (VEP), incorporating loss-of-function prediction scores for PolyPhen2 and SIFT. Afterward, the information was imported into an in-house MySQL database for further annotation, reconciliation, and data analysis by complex database queries if required.

Somatic calls were the output from MuTect (Cibulskis, Lawrence et al. 2013, Nat Biotechnol 31(3): 213-219) and VarScan (Koboldt, Zhang et al. 2012, Genome Res 22(3): 568-576). For VarScan2 results, false-positive filtering was used as indicated by the author. In order to increase the reliability of the results, only calls having at least a 9% difference in allele frequency between tumour and normal samples were considered for further analysis. Cancer-related genes were singled-out by using the information from the Catalogue of Somatic Mutations in Cancer (COSMIC) (Forbes, Beare et al. 2015, Nucleic Acids Res 43(Database issue): D805-811; Forbes, Beare et al. 2017, Nucleic Acids Res 45(D1): D777-D783.) after having translated the cancer gene consensus from COSMIC by making use of Ensembl's BioMart (Smedley, Haider et al. 2015, Nucleic Acids Res 43(W1): W589-598).

### Statistical analysis

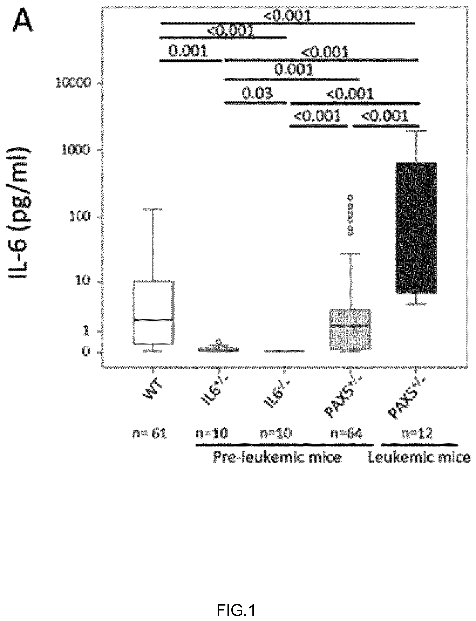

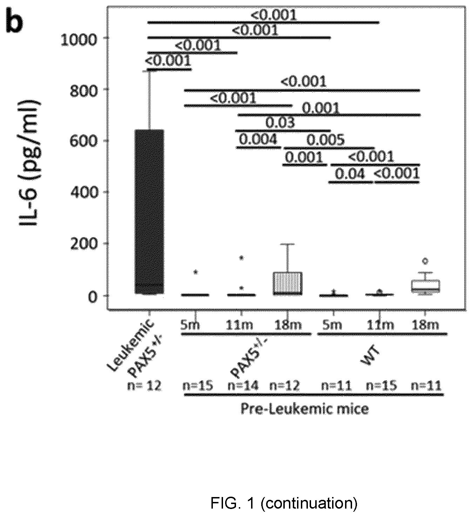

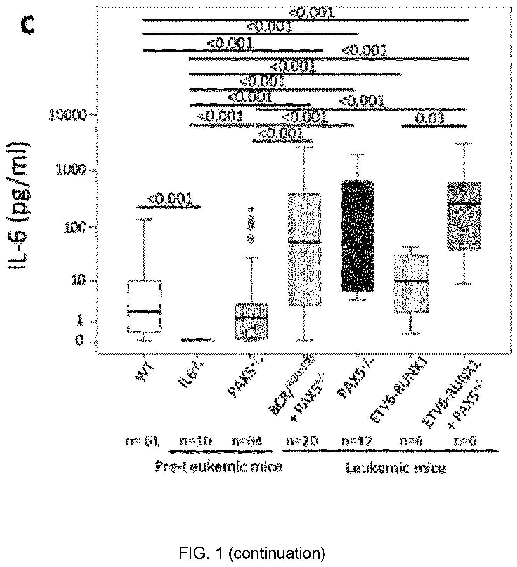

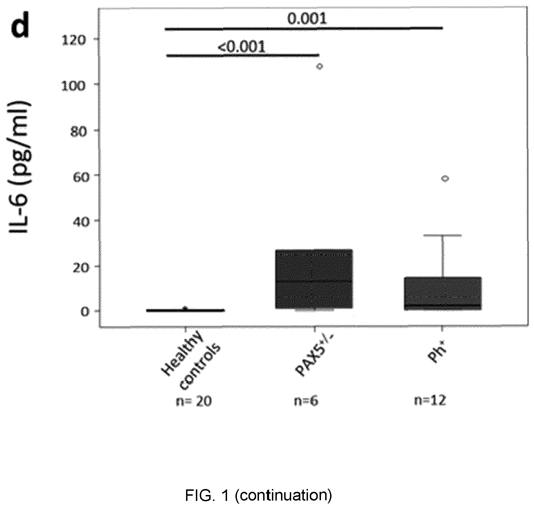

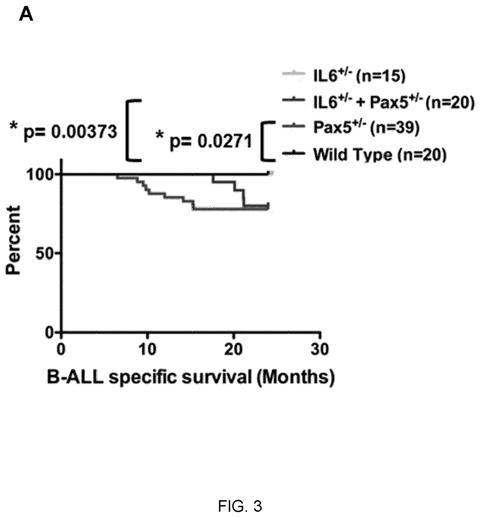

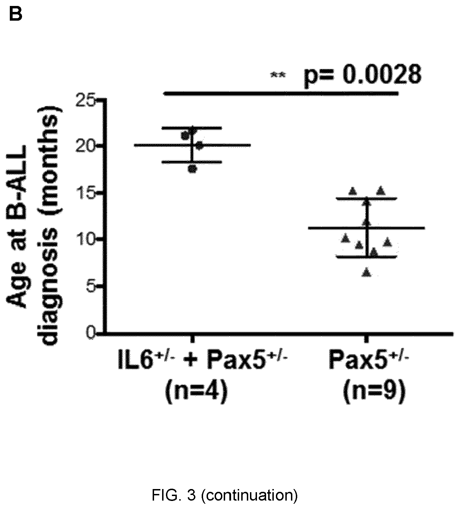

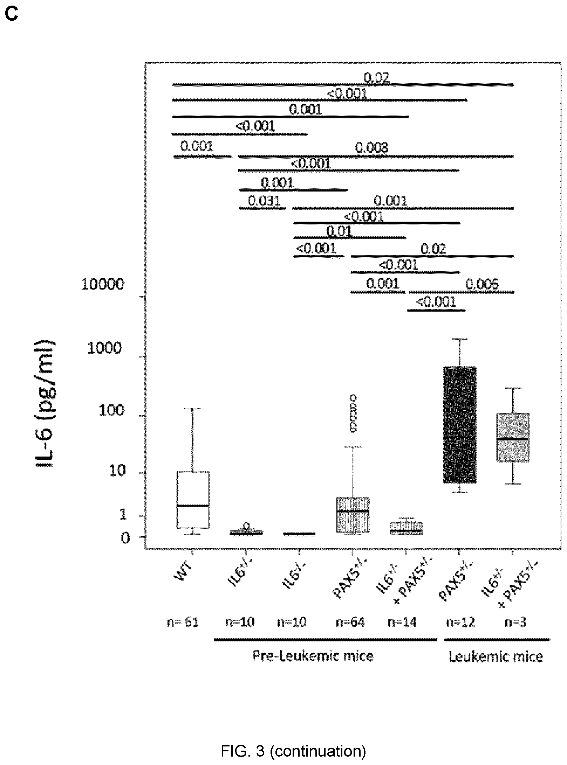

The comparisons for the average values between two sample groups were made by two-tailed Student's *t*-test or Wilcoxon's rank-sum test with GraphPad Prism software or R software. Differences in Kaplan-Meier survival plots of transgenic and WT mice were analysed using the log rank (Mantel-Cox) test. For *in vivo* transplantation experiments, the minimal number of mice in each group was calculated through use of the 'cpower' function in the R/Hmisc package. The level of significance was set at *P*<0.05. Notched boxes as represented in Figures 1 and 3 extend from the 25th to the 75th percentile values; the lines in the middle and vertical lines correspond to median values and the 10th and 90th percentiles, respectively. The Kruskal-Wallis test was used to interpret differences.

### II. RESULTS

### Pax5+/- mice developing B-ALL show enhanced expression of the proinflammatory cytokine IL-6

To determine whether the transformation of preleukemic B-cells is in part due to dysregulated expression of inflammatory cytokines in *Pax5+*/*-* 99 mice, the concentrations of 7 inflammatory cytokines (IL-2, IL-4, IL-6, IL-10, IL-17a, TNF and IFNγ) were measured in the plasma of *Pax5+*/*-* mice who developed B-ALL and age-matched control wild type mice. It was found that leukemic *Pax5+*/*-* mice had abnormal concentrations of IL-6, one of the seven measured inflammatory markers (Fig. 1a). The emergence of this increase in IL-6 levels could further be linked to disease onset, since IL-6 in plasma samples taken at routine intervals confirmed lack of IL-6 increase in *Pax5+*/*-* mice that never develop B-ALL (Fig. 1a-b). However, IL-6 increase was detectable prior to other phenotypic signs of illness in *Pax5+*/*-* mice with B-ALL (Fig. 1b). In addition, this increase in IL-6 was not observed in mouse models where the appearance of B-ALL is caused by infection exposure but not linked to a *Pax5* alteration (Fig. 1c). These elevated levels of IL-6 protein could be recapitulated at the time of diagnosis in the serum of human B-ALL carrying *PAX5* alterations (Fig. 1d). Thus, induction of a leukemogenic state by *Pax5*-loss is associated with an increase in IL-6 secretion in both human patients and mouse models, suggesting the IL-6 secretion may correspondingly be the product of oncogenic *Pax5* inactivation.

### Pax5 controls IL-6 expression in B-cells

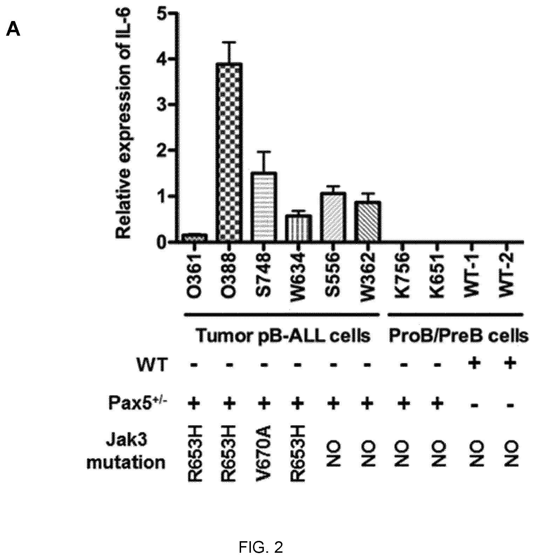

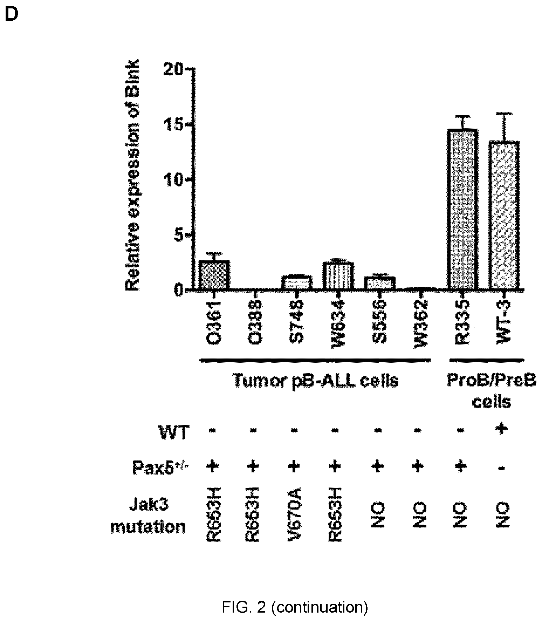

The mechanism controlling IL-6 production was investigated using leukemic proB-cells lacking *Pax5* activity and both control wild-type and *Pax5+*/*-* proB-cells. The total bone marrow of a WT mouse was used as a reference. Leukemic proB-cells lacking *Pax5* activity displayed high levels of IL-6 mRNA, which was not detectable neither in control wild-type proB-cells nor in control *Pax5+*/*-* proB-cells (Fig. 2a). Error bars represent the mean +/- the standard deviation of 3 replicates.

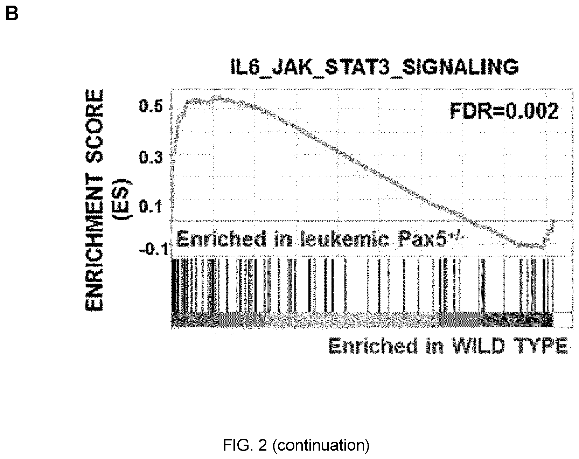

For leukemic proB-cells, analysis of gene expression confirmed enrichment of IL-6 signaling pathway (Fig. 2b) as well as enrichment in inflammatory response and apoptosis gene sets (data not shown).

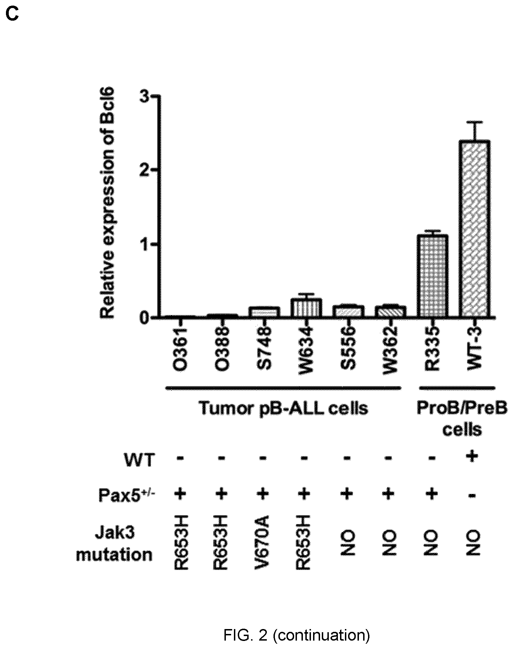

Leukemic proB-cells lacking Pax5 activity were then investigated for expression of a panel of effectors genes known to regulate IL-6 expression, and a significant downregulation of both Blnk and Bcl6 expression was found (Fig. 2c-d). Error bars represent the mean +/- the standard deviation of 3 replicates.

BCL6 is a direct transcriptional repressor 127 of the IL-6 gene (Yu et al. 2005 Blood 105, 1777-1784) and recent work has shown that STAT5 activation inhibits BCL6 expression (Duy, Hurtz et al. 2011, Nature 473(7347): 384-388). Therefore, Pax5-deficient leukemic proB-cells had decreased Bcl6 levels and this, together with the STAT5 activation, could contribute to the upregulation of IL-6 expression observed in Pax5-deficient leukemic proB-cells. These results indicate that Pax5 activity controls IL-6 expression in proB-cells, and suggest that both Bcl6 and STAT5 activation are involved in the complex molecular network mediating this effect. Taken together, these data suggest that IL-6 might be important for Pax5-deficient B-ALL development. Thus, it was then examined whether IL-6 plays any role in Pax5-mediated B-cell leukemogenesis.

### Impairment of IL-6 signaling in Pax5+/- mice delays natural infection-driven B-ALL development

Given the observed upregulation of IL-6 in *Pax5*-deficient leukemic proB-cells and mice, the requirement for IL-6 in *Pax5*-loss mediated leukemia growth in a system that recapitulates the spontaneous process of leukemogenesis was tested.

To functionally demonstrate the role of IL-6, the expression of IL-6 was impaired by breeding *Pax5+*/*-* mice) to *IL-6*+/*-* mice, which have significantly lower IL-6 serum concentrations (Fig. 1a), with the aim of testing whether *IL-6+*/*-, Pax5+*/*-* mice are resistant to infection-induced B-ALL, contrary to *Pax5+*/*-* mice.

Accordingly, control *IL-6+*/*+, Pax5+*/*-* and experimental *IL-6+*/*-, Pax5+*/*-* mice were exposed to natural infections, and B-ALL development was monitored as previously described (Martin-Lorenzo, Hauer et al. 2015, Cancer Discov., 5(12): 1328-1343.). The results showed that the reduction on IL-6 levels mitigated the emergency of leukemias in *Pax5+*/*-* mice upon exposure to natural infections (Fig. 3). B-ALL appeared between 6 and 16 months of age in *IL-6+*/*+, Pax5+*/*-* mice (mean =11.29 months) and, at the end of the 2-year experimental period, 22% of the mice had developed B-ALL (Fig. 3a-b), in line with previously reported results. In sharp contrast, B-ALL was significantly delayed in the *IL-6+*/*-, Pax5+*/*-* mice, appearing between 18 and 22 months of age (mean =20.13 months) (Fig. 3a-b), although by the termination of the experiment a similar percentage (20%) of the mice had developed. Error bars in the figures represent the mean and SD; for the significant differences, unpaired t-test p-values are indicated.

B-ALL and IL-6 was elevated in their serum similarly to *IL-6+*/*+, Pax5+*/*-* leukemias (Fig. 3c). These *IL-6+*/*-, Pax5+*/*-* B-ALLs are histologically, phenotypically and genetically similar to *IL-6+*/*+, Pax5+*/*-* B-ALLs, and FACS analyses revealed a CD19*+*/*-*B220+IgM-cKit*+*/*-*CD25+/- cell surface phenotype for tumour cells that extended through bone marrow (BM), PB, spleen and lymph nodes and infiltrated non-lymphoid tissues like liver and intestine (data not shown). All *IL-6+l-, Pax5+*/*-* B-ALLs displayed clonal immature BCR rearrangements.

The global expression signature of *IL-6+*/*-, Pax5+*/*-* B-ALLs was then characterized and compared with the expression signature of both healthy WT pro-B/pre-B-cells and

*IL-6+*/*+, Pax5+*/*-* leukemias. The analysis showed a similar differential gene expression profile (FDR=0.05) between expression patterns in *IL-6+*/*-, Pax5+*/*-* B-ALL, and *Pax5+*/*-* 167 B-ALL with just 196 probe-sets differentially expressed in contrast to the huge differences in terms of gene expression between IL-6*+*/*-*, *Pax5+*/*-* B-ALL and healthy WT proB-cells B) with 9 probe-sets differentially expressed (data not shown). Preleukemic *IL-6+*/*-, Pax5+*/*-* littermates presented a significantly reduced amount of total B-cells in the peripheral blood (PB) when compared to WT littermates of the same breeding (data not shown), but this PB B-cell decrease was similar to the one observed in *IL-6+*/*+, Pax5+*/*-* mice (data not shown).

In order to further identify somatically acquired second hits leading to leukemia development, whole exome sequencing of 4 *IL-6+*/*-, Pax5+*/*-* tumours and corresponding germline was performed on a HiSeq 2500 (Illumina) platform. *IL-6+*/*-, Pax5+*/*- tumour* DNA was derived from leukemic BM, while tail DNA of the respective mouse was used as reference germline material. Tumour-specific somatic mutations were determined by *mutect* and *varscan* analysis and the number of somatic cancer genes was calculated by using the cancer gene consensus list.

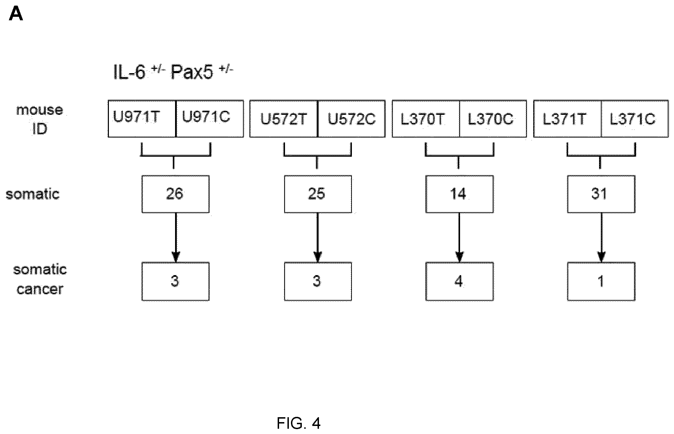

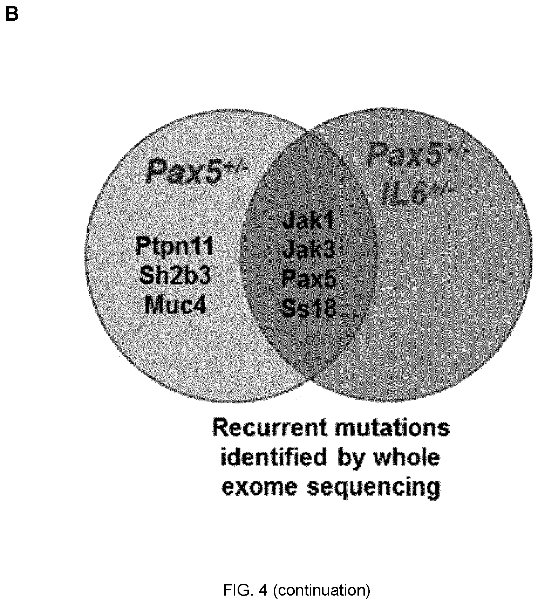

Similar to *IL-6+*/*+, Pax5+*/*-* leukemias, *IL-6+*/*-, Pax5+*/*-* tumours showed recurrent mutations affecting *Pax5, Ss18, Jak1,* and *Jak3* (Fig. 4). Taken together, these data demonstrate that the decrease of IL-6 delays spontaneous formation of infection-driven B-ALL in *Pax5+*/*-* mice. Collectively, the data suggests that knocking down amplified and clinically relevant IL-6 represents a formidable barrier to *Pax5-*dependent leukemogenesis.

### IL-6 inhibition therapeutically targets Pax5 mutant B-ALL in vivo

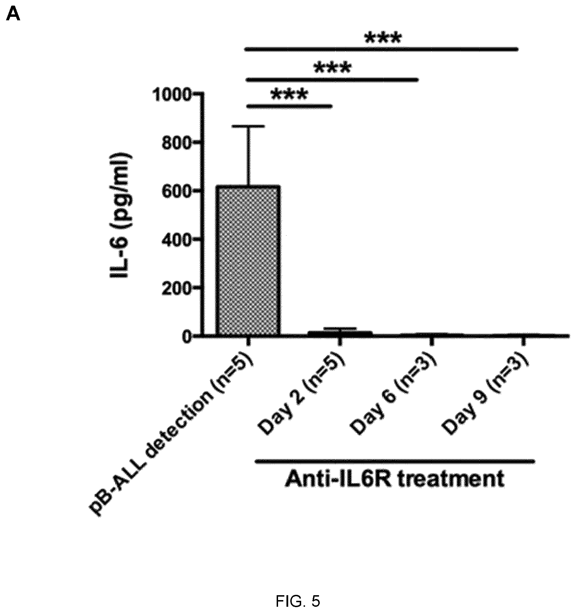

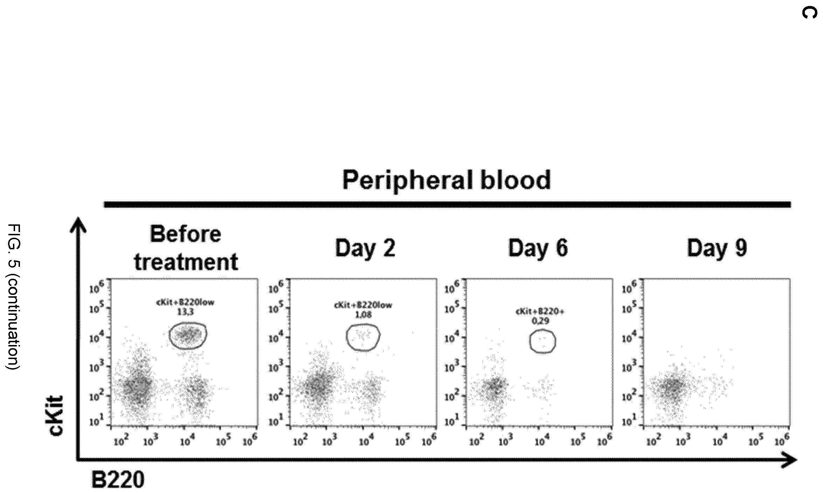

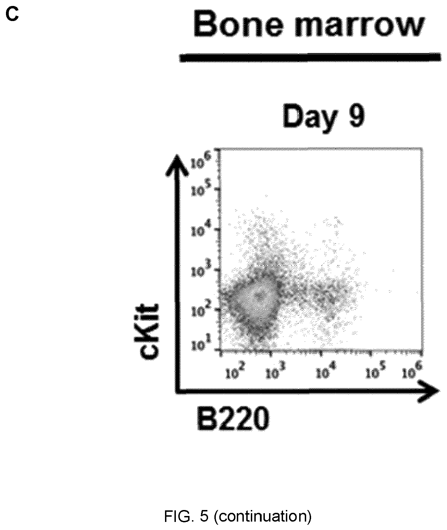

Previous results indicate that a complete understanding of how the modulation of the inflammatory signalling affects *Pax5* mutant B-ALL can only come from the analysis of intact, unmanipulated animals. Thus, it was explored if the blockade of IL-6 can modify the course of a native non-transplant *Pax5*-dependent B-ALL disease. To this aim, *IL-6*+/+, *Pax5+*/*-* mice were randomized to treatment with either immunoglobulin G (IgG) control antibody or an anti-IL-6 antibody, twice a week (10 mg/kg) once the B-ALL disease appeared as a result of natural infection exposure (Martin-Lorenzo, Hauer et al. 2015 Cancer Discov 5(12): 1328-1343.), as confirmed by the presence of blast cells in the PB (data not shown). It was found that the anti-IL-6 antibodies abolished the increase in IL-6 serum levels characteristic of *Pax5*-dependent B-ALL (Fig. 5a). Error bars represent the mean and SD; for the significant differences, an unpaired t-test was used (***); p-value <0.0001).

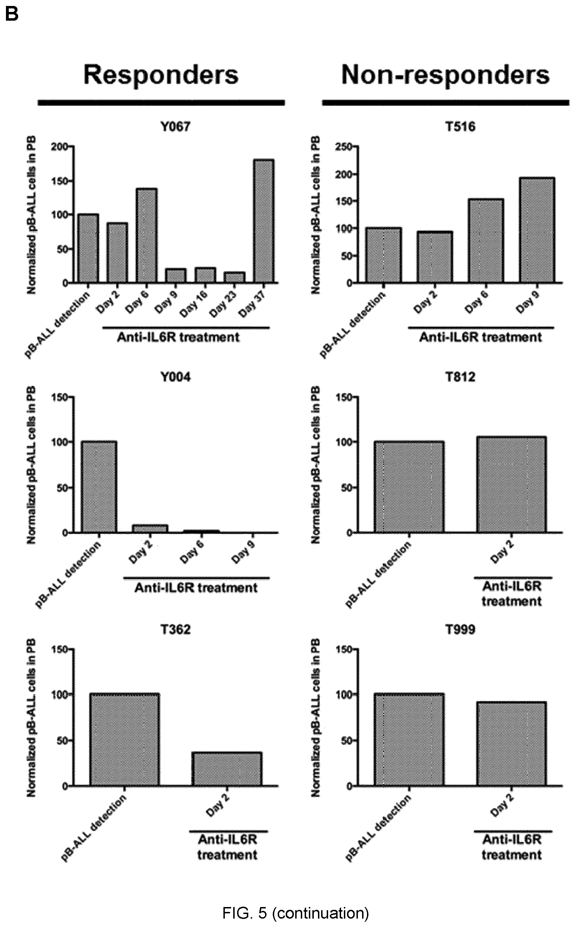

Blockade of IL-6 *in vivo* reduced disease progression in 100% of the anti-IL-6-treated mice when the percentage of blast cells in PB was lower than 40% at the time of treatment (Fig. 5b-c). However, disease progression could not be modified by IL-6 inhibition when the percentage of blast cells in PB was higher than 75% at the time of treatment (data not shown). In order to further clarify if the relapse after IL-6 inhibition is driven by pre-existing leukemic cells or by the selection of IL6-resistant clones through the acquisition of new mutations, whole exome sequencing of the 6 *IL-6+*/*+, Pax5+*/*-* tumours before anti-IL-6-treatment and after relapse was performed. Leukemias at relapse showed similar recurrent mutations than leukemias before anti-IL-6-treatment (data not shown). Thus, these data further suggest that *IL-6* retains driver functions in established leukemia and demonstrate the application of this *in vivo* native assay to identify the importance of players relevant for B-ALL development. These results show that IL-6-neutralizing antibodies may be useful therapeutic options in the treatment of *Pax5*-driven leukemias.

### III. CONCLUSION

The present results uncover an essential mechanism involving the proinflammatory cytokine IL-6 in sustaining *Pax5*-dependent B-ALL development. The results identify IL-6 as a key cytokine whose expression and secretion by mouse leukemic B-cells is induced when *Pax5* is lost during the course of B-ALL development. Impairment of IL-6 signalling delays B-ALL development, confirming the essential contribution of this pathway as a feedback loop supporting B-ALL development.

## Claims

1. An Interleukin 6 (IL-6) inhibitor for use in the treatment and/or prevention of hematologic malignancies comprising deficiencies in the B-cell transcription factor gene PAX5 in a subject.

2. The IL-6 inhibitor for use according to claim 1, wherein the hematologic malignancy is leukemia.

3. The IL-6 inhibitor for use according to claim 2, wherein the leukemia is an acute lymphoblastic leukemia.

4. The IL-6 inhibitor for use according to any one of claims 1 to 3, wherein the IL-6 inhibitor is selected from the group consisting of:
(i) an anti-IL-6 antibody or fragment thereof;
(ii) an IL-6 receptor antibody or fragment thereof;
(iii) a small molecule; and
(iv) an inhibitor of IL-6 gene expression.

5. The IL-6 inhibitor for use according to claim 4, wherein the anti-IL-6 antibody or fragment thereof is siltuximab or a fragment thereof; the IL-6 receptor antibody or fragment thereof is selected from the group comprising tocilizumab or fragment thereof, sarilumab or fragment thereof, or BCD-089 or fragment thereof; and the inhibitor of IL-6 gene expression is a siRNA, an antisense oligonucleotide, a nuclease or a ribozyme.

6. The IL-6 inhibitor for use according to any one of claims 1 to 5, wherein the IL-6 inhibitor is comprised within a pharmaceutical composition in a therapeutically effective amount.

7. The IL-6 inhibitor for use according to claim 6, wherein the pharmaceutical composition further comprises a compound for the treatment and/or prevention of hematologic malignancies in a subject.

8. The IL-6 inhibitor for use according to any one of claims 1 to 7, wherein the subject is a human subject.

9. The IL-6 inhibitor according to any one of claims 1 to 8, wherein the deficiencies in the B-cell transcription factor gene PAX5 comprise gene somatic lesions.

10. An *in vitro* method for diagnosing hematologic malignancies in a subject, or for designing a personalized therapy for a subject with hematologic malignancies, comprising
(i) determining the expression levels of the gene encoding IL-6 in a biological sample from the subject, and
(ii) comparing the expression levels obtained in step (i) with a reference level, wherein if the expression levels of said gene are increased with respect to the reference level, then the subject is suffering from a hematologic malignancy or is susceptible to receive a therapy based on an IL-6 inhibitor.

11. An *in vitro* method according to claim 10, wherein the hematologic malignancies comprise deficiencies in the B-cell transcription factor gene PAX5.

12. An *in vitro* method according to claim 10 or 11, wherein the IL-6 inhibitor is selected from the group consisting of:
(i) an IL-6 antibody or fragment thereof;
(ii) an IL-6 receptor antibody or fragment thereof;
(iii) a small molecule; and
(iv) an inhibitor of IL-6 gene expression.

13. An *in vitro* method according to claim 12, wherein the anti-IL-6 antibody or fragment thereof is siltuximab or a fragment thereof; the IL-6 receptor antibody or fragment thereof is selected from the group comprising tocilizumab or fragment thereof, sarilumab or fragment thereof, or BCD-089 or fragment thereof; and the IL-6 inhibitor is a siRNA, an antisense oligonucleotide, a nuclease or a ribozyme.

14. An *in vitro* method according to any one of claims 10 to 13, wherein the subject is a human subject.

15. An *in vitro* method according to any one of claims 10 to 14, wherein the hematologic malignancy is leukemia, preferably, the leukemia is an acute lymphoblastic leukemia.
